# EUROPEAN PATENT SPECIFICATION

(11) **EP 2 718 311 B1**
(45) Date of publication and mention of the grant of the patent: **17.01.2018**
(21) Application number: 12727917.2
(22) Date of filing: 13.06.2012
(51) Int. Cl.: C07K 14/52, A61M 1/36, C07K 17/02, G01N 33/68

(54) **TREATING MULTIPLE SCLEROSIS**
BEHANDLUNG VON MULTIPLER SKLEROSE
TRAITEMENT DE LA SCLÉROSE EN PLAQUES

(30) Priority: 13.06.2011 US 201161496264 P
(43) Date of publication of application: 16.04.2014
(73) Proprietor: TLA Targeted Immunotherapies AB, 171 76 Stockholm (SE)
(72) Inventor: COTTON, Graham, Edinburgh EH13 9PH (GB); WINQVIST, Ola, S-756 53 Uppsala (SE)
(74) Representative: Zacco Denmark A/S
(86) International application number: PCT/GB2012/051352
(87) International publication number: WO 2012/172342

(56) References cited:
- EP-A1- 1 783 227
- WO-A1-2005/037305
- WO-A1-2007/133147
- WO-A1-2010/103517
- WO-A2-2010/029317
- DE-A1-102005 036 505
- US-A1- 2003 215 421
- M. N. SARAFI ET AL: "Murine Monocyte Chemoattractant Protein (MCP)-5: A Novel CC Chemokine That Is a Structural and Functional Homologue of Human MCP-1", JOURNAL OF EXPERIMENTAL MEDICINE, vol. 185, no. 1, 1 January 1997 (1997-01-01), pages 99-110, XP055035882, ISSN: 0022-1007, DOI: 10.1084/jem.185.1.99
- CARLA IARLORI ET AL: "Interferon beta-1b modulates MCP-1 expression and production in relapsing-remitting multiple sclerosis.", JOURNAL OF NEUROIMMUNOLOGY, vol. 123, no. 1-2, 1 February 2002 (2002-02-01), pages 170-179, XP055037172, ISSN: 0165-5728
- NAKAJIMA: "Increased intrathecal chemokine receptor CCR2 expression in multiple sclerosis", BIOMARKER INSIGHTS, vol. 2, 1 January 2007 (2007-01-01), page 463, XP055037155, ISSN: 1177-2719
- NAKANE SHUNYA ET AL: "Cytapheresis with a filter for selective removal of CD4+ T cells in experimental autoimmune encephalomyelitis", MULTIPLE SCLEROSIS JOURNAL (MSJ), SAGE PUBLICATIONS, BASINGSTOKE, GB, vol. 9, no. 6, 1 December 2003 (2003-12-01), pages 579-584, XP008182114, ISSN: 1352-4585, DOI: 10.1191/1352458503MS968OA
- MAHAD DON J ET AL: "The role of MCP-1 (CCL2) and CCR2 in multiple sclerosis and experimental autoimmune encephalomyelitis (EAE)", SEMINARS IN IMMUNOLOGY, W.B. SAUNDERS COMPANY, PA, US, vol. 15, no. 1, 1 February 2003 (2003-02-01), pages 23-32, XP009139505, ISSN: 1044-5323
- PRENDERGAST CATRIONA T ET AL: "Immune Cell Entry to Central Nervous System - Current Understanding and Prospective Therapeutic Targets", ENDOCRINE METABOLIC & IMMUNE DISORDERS-DRUG TARGETS, vol. 9, no. 4, December 2009 (2009-12), pages 315-327, XP008182142, ISSN: 1871-5303
- PEASE JAMES ET AL: "Chemokine Receptor Antagonists", JOURNAL OF MEDICINAL CHEMISTRY, vol. 55, no. 22, 22 November 2012 (2012-11-22), pages 9363-9392, ISSN: 0022-2623(print)

## Description

### FIELD OF THE INVENTION

The various embodiments of the present invention relate to products for and methods of treating multiple sclerosis, in particular active and stable relapsing-remitting multiple sclerosis, primary progressive, secondary progressive and progressive relapsing multiple sclerosis. Companion diagnostics are also described.

### BACKGROUND OF THE INVENTION

Multiple sclerosis is a neurodegenerative disease affecting the central nervous system (CNS). Disease onset usually occurs in young adults between the ages of 20 and 40, however, the precise underlying cause of MS is unknown. There is currently no cure for this disabling disease and treatment is primarily focussed on management of symptoms. Irrespective of disease etiology, the immune system has been found to play a central role in the pathogenesis of MS. In particular, the lesions that develop in the brain and/or spinal cord during disease progression are frequently characterised by an excessive inflammatory infiltrate and the presence of autoreactive CD4+/CD8+ T cells and autoreactive B cells. Furthermore, ongoing assault of the CNS mediated by a variety of inflammatory and/or immune cell types appears to be the primary cause of the nerve damage and in particular, the axon demyelination, associated with this disease.
Apheresis is a treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. WO2010/029317 describes apheresis columns useful for treating inflammatory conditions including a chemokine immobilised on a solid support.

Related art is e.g. Nakane Shunya et al., Multiple Sclerosis Journal, 2003, 9(6), pp. 579-584, which relates to cytapheresis with a filter for selective removal of CD4⁺ Tcells in an experimental animal model of human multiple sclerosis.
Moreover, Mahad Don, et al., Seminars in Immunology, 2003, 15(1), pp. 23-32, relates to the role of MCP-1(CCL2) and CCR2 in multiple sclerosis and the experimental model EAE.

### SUMMARY OF THE INVENTION

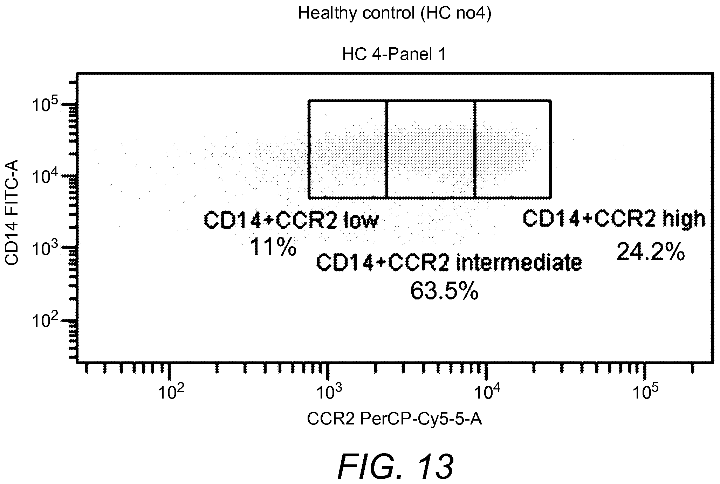

Chemokines are a class of cytokine molecules involved in cell recruitment and activation in inflammation. Chemokines cause chemotaxis and activation of various subpopulations of cells in the immune system. The activity of chemokines is mediated primarily through tight binding to their receptors on the surface of leukocytes. In certain embodiments the present invention is based on the realisation that the interaction between chemokines and cells expressing their receptors may be exploited for the treatment of multiple sclerosis. In particular, various types of multiple sclerosis, such as active and stable relapsing-remitting multiple sclerosis include an inflammatory component. The inventors have determined that targeting increased recruitment of specific chemokine receptor-expressing cells to the site of inflammation presents a new therapeutic approach to treat such conditions. Moreover, in such conditions, chemokine receptor expression on each cell may be increased again providing a therapeutic approach to treat such conditions. It is shown herein that subjects suffering from MS exhibit increased frequency of chemokine receptor expressing cells in the peripheral blood, in particular CCR2 and CCR6 expressing T lymphocytes, compared to healthy controls. It is also shown herein that the CCR2 cells can be removed using a suitable binding reagent, in particular MCP-1 (in biotinylated form) immobilized on a suitable matrix. Similarly, it is shown herein that (the additional) CCR6-expressing cells can be depleted using a suitable binding reagent, in particular CCL20 (MIP-31), in biotinylated form, immobilized on a suitable matrix.
Consequently, present invention relates to a binding reagent capable of specifically binding to the chemokine receptor CCR2, for use in the treatment of multiple sclerosis, wherein the binding reagent is immobilized on a solid support contained within an apheresis column, to which is applied peripheral blood from a patient thus removing CCR2 expressing cells from the peripheral blood of the patient, wherein the binding reagent is the MCP-1/CCL2 chemokine.
Moreover, the invention also relates to a method of selecting a suitable treatment for multiple sclerosis comprising determining
a) the levels of one or more of the chemokine receptor CCR2 expressing cells
b) levels of expression of CCR2; and/or
c) levels of cells with high expression of CCR2
in a sample obtained from a subject, wherein high levels of CCR2 expressing cells, high levels of expression of CCR2 or high levels of cells with high expression of CCR2 or increased levels of CCR2 expressing cells compared to control, increased levels of expression of CCR2 compared to a control or increased levels of cells with high expression of CCR2 compared to a control, result in selection of a treatment as defined above for use in treatment of the multiple sclerosis.
Thus, in certain embodiments the invention serves to reduce the recruitment of inflammatory leukocytes, which express characteristic chemokine receptors, and possibly express characteristic chemokine receptors at increased levels, to sites of inflammation linked to multiple sclerosis such as active and stable relapsing-remitting multiple sclerosis, primary progressive, secondary progressive and progressive relapsing multiple sclerosis. This is achieved using specific binding reagents to capture specific chemokine receptor-expressing inflammatory leukocytes from the patient. Accordingly, the invention enables a method for treating multiple sclerosis comprising applying peripheral blood from a patient to an apheresis column loaded with a solid support comprising one or more binding reagents capable of specifically binding to the chemokine receptors CCR2, immobilized directly or indirectly on the support thus removing chemokine receptor CCR2 expressing cells from the peripheral blood of the patient. The peripheral blood from which the chemokine receptor expressing cells have been removed may then be returned to the patient in order to complete the treatment.
As shown herein, suitable binding reagents can be immobilized onto a solid support, either directly or indirectly, to generate an apheresis column suitable for capturing relevant chemokine receptor-expressing cells. Where increased levels of chemokine receptor expression are observed, such cells may be preferably removed from the peripheral blood using the columns for use in the invention. Thus, the various embodiments of the invention preferably target CCR2^{hi} cells as defined herein for removal from the peripheral blood. "High" expression may be determined according to standard flow cytometry techniques. The level is measured relative to levels of expression of the chemokine receptor in cells taken from a healthy subject. The attached Figure 13 provides an example of a gating strategy. The invention provides a binding reagent capable of specifically binding to the chemokine receptor CCR2 for use in the treatment of multiple sclerosis, wherein the binding reagent is immobilized on a solid support contained within an apheresis column, to which is applied peripheral blood from a patient thus removing chemokine receptor CCR2 expressing cells from the peripheral blood of the patient, wherein the binding reagent is the MCP-1/CCL2 chemokine. Monocytes are produced by the bone marrow from haematopoietic stem cell precursors called monoblasts. Monocytes may differentiate into macrophages or dendritic cells. Monocytes and their macrophage and dendritic cell progeny serve a number of functions in the immune system including phagocytosis, antigen presentation and cytokine production. Monocytes may be characterized with reference to expression of the cell surface marker CD14, optionally together with CD16. Classical monocytes may be characterized by high level expression of the CD14 cell surface receptor (CD14++ CD16- monocyte). Non-classical monocytes may be characterized by low level expression of CD14 and with additional co-expression of the CD16 receptor (CD14+CD16++ monocyte). Intermediate monocytes may be characterized by high level expression of CD14 and low level expression of CD16 (CD14++CD16+ monocytes). Macrophages are derived from monocytes and are responsible for protecting tissues from foreign substances. They are cells that possess a large smooth nucleus, a large area of cytoplasm and internal vesicles for processing foreign material. The term "macrophage" may refer to a monocyte-derived cell expressing one or more of the following cell surface markers CD14, CD11b, Lysozyme M, MAC-1/MAC-3 and CD68. The term macrophage includes both activated and un-activated macrophages. Activated macrophages may be characterized by expression of one or more of CD69, ENG, FCER2 and IL2RA, HLA-DR, CD86. Un-activated macrophages have not yet received activating signals through for example TLR receptors and therefore they express less activation markers on the cell surface which correlates with lesser maturation. M1 macrophages may be characterized by expression of one or more of CD16⁺CD32⁺CD64⁺ and secrete mainly IL-23 and IL-1, TNF, IL-6 and high levels of IL-12 and in addition effector molecules such as iNOS and ROI. M1 macrophages have cytotoxic features as opposed to M2 macrophages. M2 macrophages may be characterized by expression of one or more of SRA/B⁺CD163⁺MR⁺CD14⁺ and express TGFβ, IL-10 and IL-1Ra. Tumour associated macrophages (TAMs) share many characteristics with the M2 macrophages and are considered as M2 polarised macrophages. The M1/M2 paradigm can also be found in monocyte subsets where CD14⁺CD16⁻CXC3R1^{low} monocytes are considered the "inflammatory" subset and the CD14^{low}CD16⁺CXC3R1^{high} are "resident" monocytes.

The three major types of lymphocyte are T cells, B cells and natural killer (NK) cells. The term "T-lymphocyte" includes CD4⁺ T cells such as T helper cells (Th1 cells and Th2 cells), and CD8⁺ T cells such as cytotoxic T cells. Th1 cells may be characterized by expression of CCR5 and/or by production of IFN-γ. Th2 cells may be characterized by expression of CCR3 and/or by production of IL-4.

The claimed invention may target eosinophils. The name "eosinophil" derives from the eosinophilic "acid-loving" properties of the cell. Normally transparent, it is this affinity that causes them to appear brick-red after staining with eosin, a red dye, using the Romanowsky method. The staining is concentrated in small granules within the cellular cytoplasm, which contain many chemical mediators, such as histamines and proteins such as eosinophil peroxidase, ribonuclease (RNase), deoxyribonucleases, lipase, plasminogen, and major basic protein. These mediators are released by a process called degranulation following activation of the eosinophil, and are toxic to both parasite and host tissues.

Eosinophils develop and mature in bone marrow. They differentiate from myeloid precursor cells in response to the cytokines interleukin 3 (IL-3), interleukin 5 (IL-5), and granulocyte macrophage colony-stimulating factor (GM-CSF). Eosinophils produce and store many secondary granule proteins prior to their exit from the bone marrow. After maturation, eosinophils circulate in blood and migrate to inflammatory sites in tissues in response to chemokines such as CCL11 (eotaxin-1), CCL24 (eotaxin-2), CCL5 (RANTES) and MCP1/4. Eosinophils may be activated by Type 2 cytokines released from a specific subset of helper T cells (Th2); IL-5, GM-CSF, and IL-3 are important for eosinophil activation as well as maturation. CD44 and CD69 have been shown to represent different types of cell-surface activation markers for human eosinophils. CD69 is absent from "fresh" eosinophils but expressed following activation (using cytokines). CD44 on the other hand is constitutively expressed but expression is significantly up-regulated in response to activation (Matsumoto et al., Am. J. Respir. Cell Mol. Biol., Volume 18, Number 6, June, 1998 860-866). Cell specific markers for eosinophils include CD9 and CDw125.

Basophils may also be known as basophil granulocyte. In contrast to eosinophils, these leukocytes are basophilic, i.e., they are susceptible to staining by basic dyes. Basophils contain large cytoplasmic granules which obscure the cell nucleus under the microscope. However, when unstained, the nucleus is visible and it usually has 2 lobes. Basophils store histamine, which is secreted by the cells upon stimulation.

Basophils have protein receptors on their cell surface that bind IgE, an immunoglobulin involved in macroparasite defense and allergy. It is the bound IgE antibody that confers a selective response of these cells to environmental substances, for example, pollen proteins or helminth antigens. Recent studies in mice suggest that basophils may also regulate the behavior of T cells and mediate the magnitude of the secondary immune response. Basophils may display an immunophenotype based upon expression (or lack thereof, indicated as "+" or "-" respectively of one or more of the following markers: FcεRI+, CD123, CD49b(DX-5)+, CD69+, Thy-1.2+, 2B4+, CD11 bdull, CD117(c-kit)-, CD24-, CD19-, CD80-, CD14-, CD23-, Ly49c-, CD122-, CD11c-, Gr-1-, NK1.1-, B220-, CD3-, γδTCR-, αβTCR-, α4 and β4-integrin negative.

When activated, basophils degranulate to release histamine, proteoglycans (e.g. heparin and chondroitin), and proteolytic enzymes (e.g. elastase and lysophospholipase). They also secrete lipid mediators like leukotrienes, and several cytokines. Histamine and proteoglycans are pre-stored in the cell's granules while the other secreted substances are newly generated. Each of these substances contributes to inflammation. Recent evidence suggests that basophils are an important source of the cytokine, interleukin-4, perhaps more important than T cells. Interleukin-4 is considered one of the critical cytokines in the development of allergies and the production of IgE antibody by the immune system. There are other substances that can activate basophils to secrete which suggests that these cells have other roles in inflammation.

The various embodiments of the claimed invention may involve specific binding interactions with cell-surface (and cell-specific) markers in addition to the removal based upon binding to CCR2.

CCR2 expressed on these aforementioned cells are bound by chemokines such as monocyte chemoattractant protein-1 (MCP-1).

MCP-1 (CCL2) is a major chemoattractant for monocytes and memory T cells by means of their binding to its specific cell-surface receptor, CC-chemokine receptor-2 (CCR2). CCR2 is the gene symbol approved by the HUGO Gene Nomenclature Committee for chemokine (C-C motif) receptor 2. The HGNC ID for this gene is 1603. The gene is located at chromosome position 3p21. The previous symbol and name for the gene is CMKBR2. Synonyms for this gene include CC-CKR-2, CD192, CKR2, FLJ78302 and MCP-1-R. The NCBI Reference Sequence is NM_001123041.2 as available on 13 June.

Treatment enabled by the invention may result in alleviation or amelioration of symptoms, prevention of progression, regression of the condition, or complete recovery. Measurable parameters of successful treatment include one or more, up to all, of Multiple Sclerosis Severity Score or MRI. In specific embodiments, a single treatment is sufficient to cause a depletion of around 10%, 20%, 30%, 40%, 50%, 60% or 70%, or higher up to 80%, 90%, 95% or more, or any range of values between and including these amounts, of the chemokine receptor CCR2 expressing cells from the peripheral blood of the patient. In specific embodiments, at least around 50% depletion is achieved in a single treatment. Thus, successful treatment may be defined with reference to depletion of CCR2 expressing cells. Treatment may lead to depletion of between approximately 100 and 500 million CCR2 expressing cells, such as monocytes, in certain embodiments and more particularly to about 100, 150, 200, 250, 300, 350, 400, 450, or 500 million CCR2 expressing cells.
By binding to the column through the binding reagent-chemokine receptor interaction, chemokine receptor expressing cells are immobilized. These immobilized cells express further unoccupied chemokine receptors, which may be of the same or different type to those used for capture. These additional chemokine receptors may permit circulating chemokines which contribute to the inflammatory condition to be captured from the peripheral blood. Thus, a reduction in circulating (specific) chemokine levels may provide a measure of successful treatment. The invention relies upon a binding reagent which is capable of specifically binding to the chemokine receptor CCR2 and which is the MCP-1/CCL2 chemokine. This specific binding reaction permits cells expressing the chemokine receptor to be removed from the peripheral blood of the patient when the blood is passed over the solid support upon or within which the binding reagent is immobilized. By "specific binding" is meant that the binding reagent displays sufficient specificity of binding and appropriate binding affinity/kinetics to permit removal of cells expressing CCR2 from the peripheral blood. Whilst it is not precluded that the binding reagent is capable of binding to other molecules, such as other chemokine receptors, the binding reagent will preferentially bind to cells expressing CCR2 and in particular to cells expressing increased levels of CCR2 (as defined further herein). Chemokines are typically, although not necessarily exclusively (particularly in the case of truncated or modified forms) agonists of their cognate receptor and serve to activate the cells expressing the relevant receptor, as would be appreciated by one skilled in the art.
In the context of the various embodiments of the present invention the term "chemokine" also comprises biotinylated or otherwise labelled chemokines. The term "chemokine" also comprises modified and truncated versions of the chemokine and chemokine fragments with the proviso that the modified or truncated form retains its ability to bind to its cognate receptor (and thus remains functional in the context of various embodiments of the the invention). The chemokine does not necessarily need to retain biological activity as it is specific binding affinity for CCR2 that is required. In certain embodiments, the chemokine lacks biological activity, for example in terms of activation of the CCR2 receptor. Modifications may be made to improve protein synthesis, for example uniformity of product and yield. As known to those skilled in the art, exemplary modifications may comprise amino acid additions, substitutions, deletions or other modifications to one or more amino acids in the chemokine. Modifications may comprise substitution of the wild type amino acid with non-natural amino acids such as norleucine (NLeu) and derivatized amino acids such as pyroglutamic acid (pyroGlu). Such modifications may be made to minimize side-product formation during storage and use of the columns of the various embodiments of the invention. Modifications may be made to improve labelling, for example inclusion of a polyethylene glycol (PEG) spacer to facilitate biotinylation. The biotinylation and/or conjugation with fluorochromes or other labelling groups of the chemokine is performed in a manner which does not substantially affect the receptor binding capacity. Site specific biotinylation or other labelling is preferred as non-selective labelling of chemokines may compromise receptor binding activity. Bioinylation or other labelling is generally preferred at or towards the C-terminus of the protein as the inventors have found that modifications in this area are generally well tolerated (in terms of a minimal effect on receptor binding capability). Biotinylation may be carried out site-specifically at any suitable amino acid. Examples of suitable amino acids include lysine, diaminopropionic acid and ornithine. Generally, reference may be made to Natarajan S et al, Int. J. Pept. Protein Res., 1992, 40, 567-74; Baumeister B, Int. J. Peptide Res. And Therapeutics, 2005, 11, 139-141; Bioconjugate techniques 2nd edition, Greg T. Hermanson.

Truncations may involve deletion of either N or C terminal amino acids as appropriate, or both. Typically, the truncated version will retain the residues required for the chemokine to fold correctly, for example to retain a chemokine fold structure, consistent with the requirement that a truncated version must retain the ability to bind to the relevant receptor (expressed by (on the surface of) a leukocyte). Chemokine molecules typically include disulphide bonds between the 1^{st} and 3^{rd} and 2^{nd} and 4^{th} cysteine residues respectively, as would be understood by one skilled in the art. Where sequences are presented herein, it is assumed that these disulphide bonds will form in the folded protein (unless otherwise stated). Truncated versions may comprise anywhere between 1 and 100 less amino acids, such as 1, 2, 3, 4, 5 etc amino acids, than the wild type amino acid sequence in certain embodiments. Of course, truncated versions may comprise further modification as detailed herein. The modified or truncated version may have 40%, 45%, 50%, 55%, 60%, 65%, 70%, 75%, 80%, 85%, 90%, 95% or more overall amino acid sequence identity with the full length wild type chemokine (where a deletion is counted as a difference in amino acid sequence) in certain embodiments. Over the common sequence between the molecules (i.e the amino acids that have not been deleted), there may be 80%, 85%, 90%, 91%, 92%, 93%, 94%, 95%, 96%, 97%, 98% or 99% amino acid sequence identity in certain embodiments. Sequence identity may be determined using known algorithms, such as BLAST or GAP analysis (GCG Program) (applying default settings), which are freely available. Chemokines may lack the N-terminal signal peptide which is cleaved off during synthesis *in vivo.*

Specific chemokines useful in the various embodiments of the present invention is MCP-1. Both MCP-1 binds solely to the chemokine receptor CCR2 and so this chemokine is preferred.

The modified and truncated MCP-1/CCL2 chemokines described in greater detail herein (with reference to the relevant amino acid sequences, as set forth in the SEQ ID NOs and accompanying experimental examples) may each be applied according to the present invention. Such modified forms may instruct the skilled person regarding additional modified forms of the same and other chemokines which may be suitable for use in the invention. Chemokines show variable sequence homology varying from less than 20% to over 90% but all share very similar tertiary structures consisting of a disordered N-terminus, followed by a long loop (the N-loop) that ends in a 3₁₀ helix, a 3-stranded β-sheet and a C-terminal helix. The overlall topology is stabilsed by disulphide bonds. This common tertiary structure is a common feature of the chemokine protein family (Fernandez EJ annd Lolis E., Annu. Rev. Pharmacol. Toxicol., 202, 42, 469-99; Allen SJ et al, Annu. Rev. Immunol., 2007, 25, 787-820,).
Truncations within this N-terminal region can maintain binding to the receptor but can lead to a change or loss of function (for examples Zhang YJ et al, J. Biol. Chem., 1994, 269, 15918, ; Gong J-H and Clark-Lewis I., J. Exp. Med., 1995, 181, 631-640; Fernandez EJ annd Lolis E., Annu. Rev. Pharmacol. Toxicol., 202, 42, 469-99; Allen SJ et al, Annu. Rev. Immunol., 2007, 25, 787-820).
Truncations at the C-terminus of the chemokine can also be made and maintain receptor binding activity (Treating Inflammatory Disorders, Ola Winqvist and Graham Cotton, WO2010/029317).
Chemokines are known by those skilled in the art to share specific receptor binding domains, including a similar monomeric fold, characterized, for example, by a disordered amino-terminal domain, followed by a conserved core region, consisting of the so called "N-loop," three anti-parallel β-strands, and a carboxyl-terminal α-helix. While not being bound by theory, it is believed that the chemokine-chemokine receptor interaction is a two-step mechanism, in which the core of the chemokine interacts first with a binding site formed by the extracellular domains of the receptor, while another interaction is formed between the chemokine N terminus and a second binding site on the receptor in order to trigger receptor activation. Thus, a "fragment," such as a functional fragment of a chemokine is intended to mean a portion of the amino acid sequence of the protein that retains binding for its cognate receptor. The fragment may include, for example, the monomeric fold region, or portions thereof such as the amino-terminal domain, the conserved core region and/or the "N-loop," the anti-parallel β-strands, and/or the carboxyl-terminal α-helix or combinations and portions thereof.
Further, it is recognized that a polypeptide can be considerably mutated without materially altering one or more of the polypeptide's functions, for example, without altering specific binding and/or the folding of the protein. The genetic code is well known to be degenerate, and thus different codons encode the same amino acids. Even where an amino acid substitution is introduced, the mutation can be conservative and have no material impact on the essential functions of a protein (see for example, Stryer, Biochemistry 4th Ed., W. Freeman & Co., New York, NY, 1995). This includes, for example, the ability of the protein to bind and interact with other proteins, such as a truncated chemokine binding to its cognate receptor.

In some examples, part of a polypeptide chain can be deleted without impairing or eliminating all of its functions. For example, the deletion of between about 1 and about 20 amino acids on the C- and/or N-terminus, such as deletions of about 1, 2, 3, 4, 5, 6, 7, 8, 9, 10, 11, 12, 13, 14, 15, 16, 17, 18, 19, or 20 amino acids at the C- and/or N-terminus, can result in a chemokine that retains function, such as specific binding of its cognate receptor. Such truncations can retain the full function of an entire protein, and/or can allow for retained functions such as protein-protein interactions as in the case of ligand-receptor interactions. Chemokines having deletions of a small number of amino acids, for example, less than about 20% (such as less than about 18%, less than about 15%, less than about 10%, less than about 8%, less than about 5%, less than about 2%, or less than about 1 %) of the total number of amino acids in the wild type chemokine can also be used in the methods and devices disclosed herein. Moreover, insertions or additions can be made in the polypeptide chain for example, adding epitope tags, without impairing or eliminating its functions (Ausubel et al., Current Protocols in Molecular Biology, Greene Publ. Assoc. and Wiley-Intersciences, 1998). Other modifications that can be made without materially impairing one or more functions of a polypeptide include, for example, in vivo or in vitro chemical and biochemical modifications or the incorporation of unusual amino acids. In some examples, a functional fragment of a chemokine may consist of about 10 or more, about 25 or more, about 50 or more, about 75 or more, about 100 or more, about 125 or more, about 150, about 175 or more, or about more or 200 or more amino acid residues of a chemokine amino acid sequence.

In some examples, the chemokine has an amino acid that has at least about 60% or 65% sequence identity, about 70% or 75% sequence identity, about 80% or 85% sequence identity, about 90%, 91%, 92%, 93%, 94%, 95%, 96%, 97%, 98% or 99% sequence identity over its full length as compared to a reference sequence, such as those detailed herein, for example using the NCBI Blast 2.0 gapped BLAST set to default parameters. Alignment may also be performed manually by inspection. One or more conservative amino acid modifications can also be made in the chemokine amino acid sequence, whether an addition, deletion or modification, that does not substantially alter the 3-dimensional structure of the polypeptide or its ability to bind to the cognate receptor. For example, a conservative amino acid substitution does not affect the ability of the chemokine to specifically bind its cognate receptor. Conservative substitution tables providing functionally similar amino acids are well known in the art. The following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W).

Chemokines can be modified by a variety of chemical techniques to produce derivatives having essentially the same activity or function-such as binding to a cognate receptor-as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C1-C16 ester, or converted to an amide of formula NR1R2 wherein R1 and R2 are each independently H or C1-C16 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C16 alkyl or dialkyl amino or further converted to an amide.

Hydroxyl groups of the peptide side chains can be converted to C1-C16 alkoxy or to a C1-C16 ester using well-recognized techniques. Phenyl and phenolic rings of the peptide side chains can be substituted with one or more halogen atoms, such as F, Cl, Br or I, or with C1-C16 alkyl, C1-C16 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the peptides of this disclosure to select and provide conformational constraints to the structure that result in enhanced stability. For example, a C- or N-terminal cysteine can be added to the peptide, so that when oxidized the peptide will contain a disulfide bond, generating a cyclic peptide. Other peptide cyclizing methods include the formation of thioethers and carboxyl- and amino-terminal amides and esters.

Amino acids in a peptide, polypeptide, or protein generally are chemically bound together via amide linkages (CONH). Additionally, amino acids may be bound together by other chemical bonds. For example, linkages for amino acids or amino acid analogs can include CH2NH-, - CH2S-, -CH2-CH2 -, -CH=CH-- (cis and trans), -COCH2 --, -CH(OH)CH2-, and -CHH2SO-(These and others can be found in Spatola, in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci pp. 463-468, 1980; Hudson, et al., Int J Pept Prot Res 14:177-185, 1979; Spatola et al. Life Sci 38:1243-1249, 1986; Harm J. Chem. Soc Perkin Trans. 1307-314, 1982; Almquist et al. J. Med. Chem. 23:1392-1398, 1980; Jennings-White et al. Tetrahedron Lett 23:2533, 1982; Holladay et al. Tetrahedron. Lett 24:4401-4404, 1983; and Hruby Life Sci 31:189-199, 1982.

More specifically, MCP-1 is useful for removing CCR2 expressing cells from the blood of a patient.
CCL2 is the gene symbol approved by the HUGO Gene Nomenclature Committee for chemokine (C-C motif) ligand 2, also known as MCP-1. The HGNC ID for this gene is 10618. The gene is located at chromosome position 17q11.2-q21.1. The previous symbol and name for the gene is SCYA2 "small inducible cytokine A2 (monocyte chemotatic protein 1, homologus to mouse Sig-je)". Synonyms for this gene include GDCF-2, HC11, MCP1, MGC9434, SMC-CF, "monocyte chemoattractant protein-1", "monocyte chemotactic and activating factor", "monocyte chemotactic protein 1, homologous to mouse Sig-je", "monocyte secretory protein JE", "small inducible cytokine subfamily A (Cys-Cys), member 2". The Genbank reference sequence for CCL2 is BC009716.1 as available on 13 June 2011. Examples of suitable modified chemokines of the various embodiments of the invention containing modifications and/or truncations and specifically adapted for use in the invention are described in detail herein. MCP-1 has been produced with residue 75, which may be a lysine (or any other amino acid such as ornithine and diaminopropionic acid, that may be biotinylated), as the site of biotinylation on the chemokine (numbering based upon the mature protein having the amino acid sequence of SEQ ID NO: 2). Biotinylation permits immobilization of MCP-1 on a solid support (via a biotin-avidin interaction). The basic amino acid sequence of MCP-1, including a 23 amino acid leader sequence is set forth as SEQ ID NO: 1. The amino acid sequence of the mature protein is set forth as SEQ ID NO: 2. The inventors have determined that chemokines may display improved binding properties where the chemokine is biotinylated via a spacer group. The spacer may prevent the biotin group from impacting on the binding affinity of the chemokine. Any suitable spacer group may be employed. Further modifications may provide the molecule with advantageous properties. The invention also relates to derivatives of truncated MCP-1 chemokines. The amino acid sequence of the truncated version is set forth as SED ID NO:3.

Accordingly, in certain embodiments the invention uses a modified MCP-1 chemokine comprising, consisting essentially of or consisting of the amino acid sequence set forth as SEQ ID NO: 1, SEQ ID NO: 2 or SEQ ID NO: 3 in which one or more of the following modifications have been made:
a) the glutamine residue 1 of SEQ ID NO: 2 has been replaced with pyroglutamine
b) the C terminus is produced as an amide derivative (this may be achieved by synthesis on an amide linker)
c) the (C terminal region) residue at position 98 of SEQ ID NO: 1 or position 75 of SEQ ID NO:2 or position 67 of SEQ ID NO: 3, which may be a lysine or ornithine residue, is biotinylated, optionally via a spacer group, in order to permit immobilization of the chemokine on a solid support; and/or
d) the methionine residue at position 87 of SEQ ID NO: 1 or position 64 of SEQ ID NO: 2 or position 56 of SEQ ID NO: 3 has been replaced with norleucine.

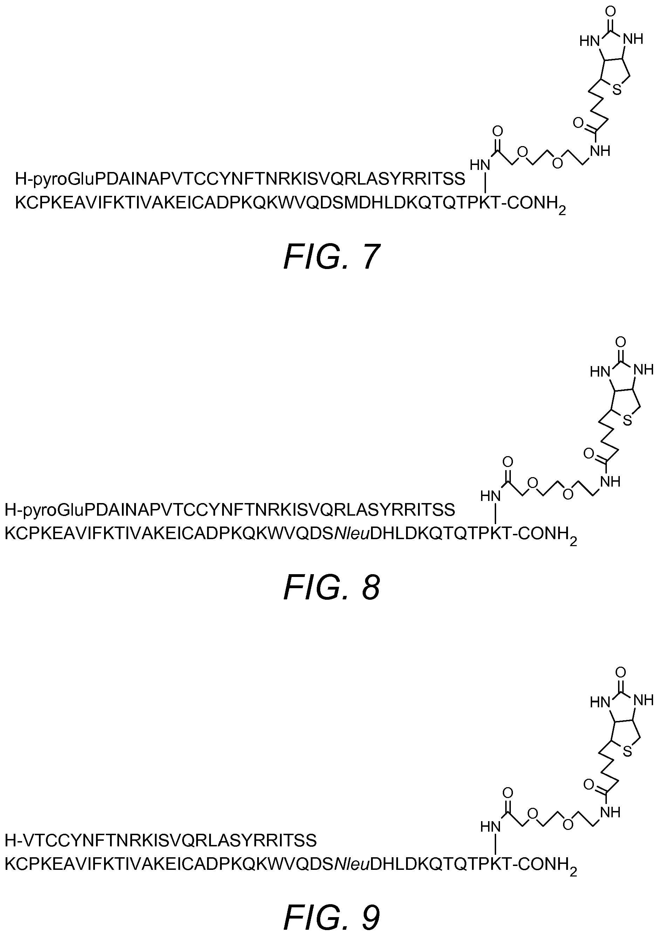

The (C terminal region) amino acid, which may be a lysine residue or a functional equivalent, at position 98 of SEQ ID NO: 1 or position 75 of SEQ ID NO:2 or position 67 of SEQ ID NO: 3 may be biotinylated via a suitable spacer group, such as a polyethylene glycol (PEG) spacer group, in order to permit immobilization of the chemokine on a solid support. In specific embodiments, the PEG spacer is 3,6-dioxo aminooctanoic acid. The sequence and biotinylation of the modified MCP-1 chemokines of the invention are shown in figures 7 to 9 respectively. The modified MCP-1 chemokines may be agonists or antagonists of CCR2 activity. They can be tested for activity in a suitable assay, such as cell-based assays. In particular, agonist and antagonist properties may be determined in functional cell-based assay on human CCR2 receptor.
The modified CCL2 (MCP-1) corresponds to residues 1 to 76 of the full length mature protein (and lacks the N-terminal signal peptide of 23 amino acids, which is cleaved off) and thus retains the chemokine fold. The Gln at the N-terminus of the protein (Gln1) is substituted with pyroglutamine to prevent mixed species of N-terminal Gln and pyroGlu being generated (SEQ ID NO: 8). This improves the yield of synthesis and ensures a homogeneous chemokine preparation through column manufacture and use. FmocLys(ivDde)-OH is incorporated as residue 75 to facilitate site-specific labelling at this position of the protein (SEQ ID NO: 9). A suitable spacer, such as a PEG spacer, may be incorporated between the ε-amino functionality and the biotin. Thus, the invention may use a modified chemokine, including a biotinylated version, which comprises, consists essentially of or consists of the amino acid sequence of SEQ ID NO: 10: X1 = pyroGlu (but may remain as Gln in some embodiments)
X75 = an amino acid residue that can be biotinylated, such as lysine, ornithine or diaminopropionic acid and optionally is biotinylated, optionally via a spacer molecule such as PEG, e.g. K(PEG-Biotin).

Chemokines useful in the various embodiments of the invention may be synthesised through any suitable means known in the art. Preferably, the chemokines are chemically synthesised as this facilitates modification and labelling etc. However, recombinant DNA based approaches may also be employed in combination with appropriate labelling and modification technologies as required.
The chemokines useful in the various embodiments of the invention can be biotinylated by methods known in the art such as described in WO 00/50088 A2. As indicated above, site-specific labelling of the chemokines of the various embodiments of the invention is preferable, although any labelling technique which does not significantly affect the receptor-binding capacity of the chemokine may be employed. Various site-specifically biotinylated chemokines and native chemokines are available commercially, for instance from Almac, Craigavon, UK. In specific embodiments the one or more chemokines are biotinylated via a spacer group. The spacer may be employed to prevent the biotin group from impacting on the activity of the chemokine, in particular binding of the chemokine to its cognate receptor. Any suitable spacer that facilitates retention of receptor binding properties of the chemokine may be employed in the various embodiments of the invention. Thus, in the specific embodiments described above, spacers other than PEG spacers may be employed as appropriate. In specific embodiments, the spacer is a polyethylene glycol (PEG) spacer. PEG has been shown to be an effective spacer permitting attachment of biotin to the chemokine (which can then be immobilized on the solid support through interaction with streptavidin) without compromising receptor binding capability.

In the context of the present disclosure the term "antibody" includes all immunoglobulins or immunoglobulin-like molecules with specific binding affinity for the relevant chemokine receptor (including by way of example and without limitation, IgA, IgD, IgE, IgG and IgM, combinations thereof, and similar molecules produced during an immune response in any vertebrate, for example, in mammals such as humans, goats, rabbits and mice). Specific immunoglobulins disclosed herein include IgG isotypes. The antibodies disclosed herein may be monoclonal or polyclonal in origin, but are typically monoclonal antibodies. Antibodies may be human antibodies, non-human antibodies, or humanized versions of non-human antibodies, or chimeric antibodies. Various techniques for antibody humanization are well established and any suitable technique may be employed. The term "antibody" also refers to a polypeptide ligand comprising at least a light chain or heavy chain immunoglobulin variable region which specifically recognizes and binds an epitope of an antigen, and it extends to all antibody derivatives and fragments that retain the ability to specifically bind to the relevant chemokine receptor. These derivative and fragments may include Fab fragments, F(ab')₂ fragments, Fv fragments, single chain antibodies, single domain antibodies, Fc fragments etc. The term antibody encompasses antibodies comprised of both heavy and light chains, but also heavy chain (only) antibodies. The antibodies may be engineered so as to be specific for more than one chemokine receptor, for example bi-specific to permit binding to two different chemokine receptors. Suitable commercially available antibodies which bind to the chemokine receptors of interest are listed in table 1 below. They may or may not be labelled. General reference may be made to "Antibodies a laboratory manual: By E Harlow and D Lane. pp 726. Cold Spring Harbor Laboratory. 1988".

**Table 1. Commercially available fluorophore labelled antibodies against specific chemokine receptors**

| **Antibody** | **Fluorophore** | **Supplier** |
|---|---|---|
| CCR5 | PE | Biolegend |
| CCR2 | PerCP Cy5.5 | Biolegend |
| CCR6 | PerCP Cy5.5 | BD Biosciences |
| CCR3 | PE | Biolegend |
| CCR1 | Alexa Fluor 647 | Biolegend |
| CCR9 | APC | R&D Systems |

Anti-CCR2 antibodies are described for example in WO 2010/021697. Further examples of potentially useful antibodies include MLN-1202, an anti-CCR2 monoclonal antibody currently undergoing clinical trials (Millennium Pharmaceuticals).
The chemokine receptor expressing cells may thus be targeted using alternative binding agents, such as antibodies or other chemical compounds, as defined herein, rather than the natural chemokine binding partner. This is however not part of the invention. This approach is a new approach to treating inflammatory conditions and in particular multiple sclerosis.

Thus, disclosed herein is an apheresis column loaded with a solid support comprising a binding reagent capable of specifically binding to a chemokine receptor immobilized directly or indirectly on the support to permit removal of a cell expressing the chemokine receptor from the peripheral blood of a patient, wherein the binding reagent is a chemokine. The binding reagent capable of specifically binding to the chemokine receptor may be an agonist or an antagonist of the chemokine receptor.

Similarly, in other aspects disclosed herein is a binding reagent capable of specifically binding to a chemokine receptor for use in the treatment of multiple sclerosis, wherein the binding reagent is immobilized on a solid support contained within an apheresis column as defined above (an apheresis column loaded with a solid support comprising a binding reagent capable of specifically binding to a chemokine receptor immobilized directly or indirectly on the support to permit removal of a cell expressing the chemokine receptor from the peripheral blood of a patient/subject, wherein the binding reagent is a chemokine), to which is applied peripheral blood from a patient thus removing chemokine receptor expressing cells from the peripheral blood of the patient.
Solid support materials for immobilizing the binding reagents of the various embodiments of the invention are known in the art. "Solid support" refers to, for example, materials having a rigid or semi-rigid surface or surfaces, and may take the form of beads, resins, gels, microspheres, or other geometric configurations. A useful support material is one that does not activate blood cells so as to make them coagulate or adhere to the support as peripheral whole blood is applied to the device. In certain embodiments, a support treated with an agent to provide it with anti-coagulation properties, in particular a heparinized support is employed. Alternatively, the blood of the patient may be treated with an anti-coagulant such as heparin prior to application to the support. Useful support materials comprise high molecular weight carbohydrates, in particular carbohydrates having a molecular weight of 100 kDa or more, such as agarose, in particulate form, optionally cross-linked, and cellulose. Other preferred support materials are polymers, such as carboxylated polystyrene, and glass. The support of the various embodiments of the invention may be provided in the form of particles or fibres. The support particles may have regular form, such as spheres or beads, or irregular form. They may be porous or non-porous. A preferred average particle size of the support is from 50 µm to 2 mm. In certain embodiments Sepharose™, a cross linked, beaded-form of agarose, is used as column matrix. It is chosen for its optimal distribution capacity and can provide a large available area for affinity binding. Solid supports may be provided in the form of magnetic beads, with the specific binding reagent immobilized on the magnetic beads. Following capture of the CCR2 chemokine receptor expressing cells from the blood, the beads can be removed from the blood with the aid of an appropriate magnetic separator.

Methods for immobilizing binding reagents on a solid support are known in the art. A binding reagent, such as a chemokine, antibody, peptide, nucleic acid or chemical compound, can be immobilized on the support in a direct or indirect manner. Immobilization can be by means of a suitable linker in some embodiments. A preferred method of indirect immobilization of a binding reagent, such as a chemokine, relies upon the interaction between biotin and avidin molecules. "Avidin" or "avidin molecule" refers to any type of protein that specifically binds biotin to the substantial exclusion of other (small) molecules that might be present in a biological sample. Examples of avidin include avidins that are naturally present in egg white, oilseed protein (e.g., soybean meal), and grain (e.g., corn/maize), and streptavidin, which is a protein of bacterial origin. Thus, biotinylation of the binding reagent and use of an avidin molecule such as streptavidin immobilized on the solid support allows reliable attachment of the binding reagent to the solid support according to methods known in the art. Specifically, such a method may comprise providing the binding reagent in biotinylated form, providing a solid support having streptavidin immobilized on its surface, contacting the support with an aqueous solution of the biotinylated binding reagent, and rinsing the support with an aqueous solvent. In addition, binding pair interactions, such as antibody - antigen interactions, may also be utilised for indirect immobilisation of binding reagent onto a support. In such embodiments the support may be derivatised with one member of a binding pair, such as an antibody or fragment or derivative thereof, as defined herein, which has known affinity for a particular peptide sequence or small molecule hapten. Incorporating the other member of the binding pair, such as an antigen, peptide sequence or the hapten onto or into the binding reagent facilitates immobilisation onto a solid support coated with the corresponding antibody or fragment or derivative thereof. Thus, the binding reagent may be modified to include the peptide sequence or hapten into the linear molecule or may be added as a side chain or label. Any suitable antibody-antigen pair may be employed. The antibody fragment or derivative may be any fragment or derivative that retains specific binding affinity for the appropriate antigen. Examples include Fab, F(ab')₂ fragments, scFV, VH domains, single domain antibodies (such as nanobodies), heavy chain antibodies and humanized version of non-human antibodies etc. Other high affinity interactions can be utilised for immobilisation of binding reagents, as long as the binding reagent can be synthesised or derivatised with one of the interacting partners and the solid support synthesised or derivatised with the other interacting partner without loss of binding activity (i.e. binding of the binding reagent to the appropriate chemokine receptor). Immobilization may occur via essentially the same interaction in reverse in some embodiments. Thus, the binding reagent which may be a chemokine for example, may be attached to an antibody as defined herein, and the solid support derivatised with the antigen. The chemokine may be produced as a fusion protein with the antibody.

Alternatively the chemokines can be immobilised directly onto a solid support using bioconjugation techniques established in the field. For example direct immobilisation of proteins onto cyanogen bromide activated solid supports via amino functionalities within the primary sequence of the protein. Alternatively, sulphydryl functionalities within proteins can be used to directly immobilise the protein to alkyl halide derivatised supports or supports containing free thiol functionalities. In further embodiments, proteins containing α-thioester functionalities can be directly immobilised on supports containing 1,2 amino thiol moieties (eg N-terminal cysteine) using the native chemical ligation reaction. Alternatively proteins modified with ketones and aldehydes can be immobilised on solid supports derivatised with hydrazinyl, hydrazide and aminoxy functionalities using hydrazone / oxime bond forming ligation reactions (and vice versa). Alternatively 'Click' chemistry can be used to immobilise proteins onto solid supports, whereby the protein and the support are derivatised with the appropriate mutually reactive chemical functionalities (azides and alkynes). In other embodiments Staudinger ligation chemistry can be used to immobilise appropriately derivatised proteins onto the appropriately derivatised solid supports.

The solid support is contained within or carried by the apheresis column. Thus, by "loaded" is meant that the column carries or contains the solid support in a manner such that (peripheral) blood can flow through the column in contact with the solid support. Thus, the solid support provides a matrix within the column through which blood flows, in continuous fashion in certain embodiments. This permits cells expressing the specific chemokine receptor to be removed from the blood passing through the column, such that blood exiting the column is depleted of the specific chemokine receptor-expressing cells. In specific embodiments, the apheresis column is loaded with a support comprising streptavidin immobilized on the support and one or more biotinylated binding reagents, such as chemokines, bound to the streptavidin on the support. The solid support may be comprised of a high-molecular weight carbohydrate, optionally cross-linked, such as agarose.

As discussed above, the binding reagent is coupled to the solid support. The relative amounts of binding reagent may be controlled to ensure that coupling between the solid support and the binding reagent will be immediate, minimising the risk of binding reagent decoupling from the solid support. Thus, it may be ensured that there is a relative excess of immobilization sites for the binding reagent on the solid support. Alternatively, or additionally, following immobilization of the binding reagent on the solid support, the solid support may be washed to remove any unbound binding reagent.

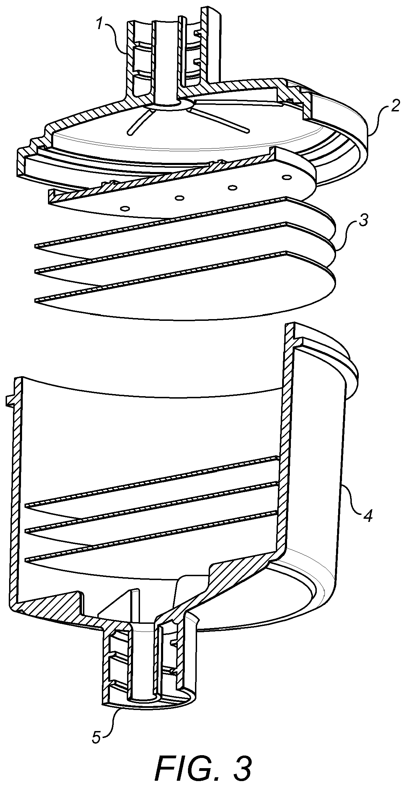

The apheresis column utilised in the various embodiments of the present invention acts as a leukapheresis treatment for conditions associated with multiple sclerosis. The column acts to specifically remove CCR2 -expressing monocytes by exploiting the interaction between CCR2 expressed on the cell surface and a specific binding reagent immobilized on a solid support contained within or carried by the column. The overall column typically comprises, consists of, or consists essentially of three combined components; 1) a housing which contains or carries 2) the solid support and 3) one or more binding reagents (immobilized thereon) which specifically bind one or more chemokine receptors. The housing may be manufactured from any suitable material for clinical use. In certain embodiments the housing is composed of a plastic material. The housing includes an in flow site for entry of blood and an out flow site for blood (depleted of target cells) to exit the column. The housing may be designed to maintain a continuous blood flow through the solid support matrix. The housing (as shown for example in Figure 3) may include a top portion which comprises a distribution plate (2) at the inflow site (1) to spread the blood evenly over the entire matrix area. The distribution plate may act as a first safety barrier preventing larger particles flowing through the column and into the patient. However, the distribution plate is not essential and may be removed in some embodiments to decrease the overall resistance in the system. The column may contain one or more safety filter units (3 and 4) placed at the inflow (1) and/or outflow (5) sites of the plastic housing. Such filter units may act to prevent particles larger than blood cells passing in and/or out of the column. The safety filter units may contain a plurality of filters, such as two, three or four filters designed to be a robust barrier and stop all particles larger than blood cells passing through the column. Inclusion of safety filters (3 and 4) at both ends of the column serves to minimize the risk of leakage of particles into the patient, including in the event that the device is incorrectly connected resulting in blood flow in the opposite direction to that intended. The safety filters may comprise of any suitable pore size to prevent particles larger than blood cells from passing through the column, as would be readily understood by one skilled in the art. Suitable filters are commercially available. In specific embodiments, the pore size of the filter(s) is between approximately 60µm and 100µm, more specifically approximately 80 µm. The solid support and binding reagent components are discussed in further detail herein.

The volume of the housing may be varied depending upon the blood volumes intended to pass through the column. Typically, the volume of the housing is between approximately 40ml and 200ml, more specifically 50ml to 150ml or 60ml to 120ml.

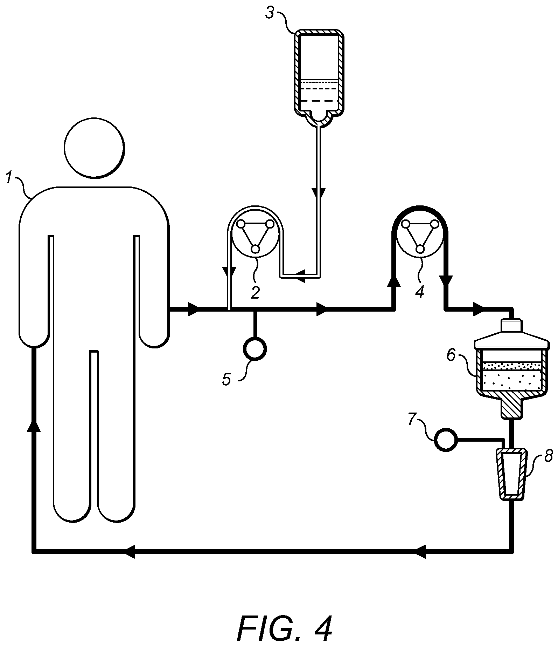

The column is generally applied in the form of an apheresis circuit. In this context, the overall system includes the apheresis column, tubing and an appropriate pump to pump the blood around the circuit. The system is illustrated in figure 4. The patient (1) is connected to the extracorporeal circuit via sterile needles to veins in the right and the left arms. A saline bag (3) is also connected and the saline solution is pumped with a suitable pump (2). Blood is drawn from one arm of the patient through the sterile tubing system by the blood pump (4) and passed through the column (6) and back to the patient. The tubing system may be connected to the column via any suitable coupling, such as standard dialysis luer-lock couplings. The couplings on the column may be colour-coded for correct assembly. For example, red tubing for inflow to the red column top and blue tubing for outflow back to the patient. An air detector (8) may be present in the circuit. Inlet pressure (5) and/or Pven sensors (7) may additionally be employed to monitor the pressure in the circuit.

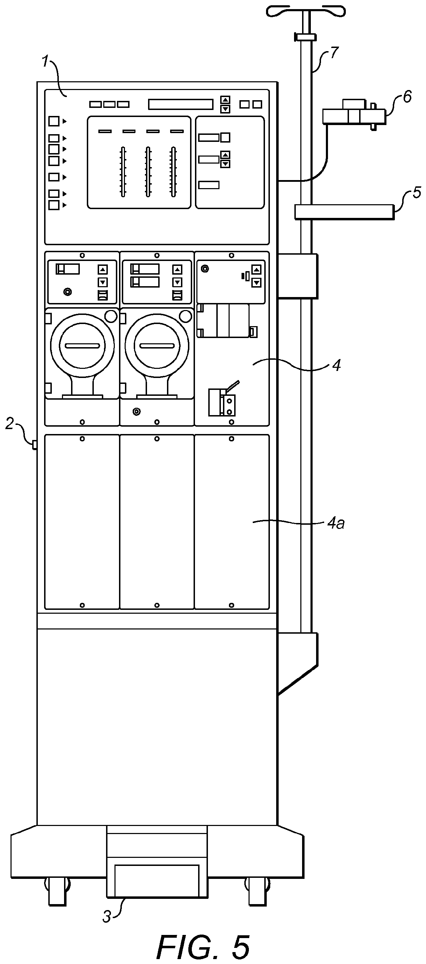

An apheresis pump, such as the 4008 ADS pump manufactured by Fresenius Medical Care or the Adamonitor pump, may monitor the patient's inflow and outflow. The pump may also monitor the pressure in the extracorporeal circulation. The pump may be able to discriminate air by a bubble catcher and air detector. A clot catcher filter may be positioned inside the bubble catcher. The pump may also incorporate an optical detector to distinguish between light, e.g. saline solution or air present in the tubing system and dark e.g. blood present in the tubing system.
A schematic diagram of a suitable pump, showing the air detector and optical filter is shown in figure 5. If the pump system detects air bubbles and optical fluctuations or if extracorporeal pressure values are out of the set range, then the pump may stop immediately. Alternatively or additionally a visual/ audible alarm may be emitted.
"Diagnosing" is defined herein to include screening for a disease/condition or pre-indication of a disease/condition, identifying a disease/condition or pre-indication of a disease/condition and checking for recurrence of disease/condition following treatment. The methods disclosed herein may also have prognostic value, and this is included within the definition of the term "diagnosis". The prognostic value of the methods disclosed herein may be used as a marker of potential susceptibility to multiple sclerosis by identifying levels of CCR2 expression linked to conditions associated with an multiple sclerosis. Thus patients at risk may be identified before the disease has a chance to manifest itself in terms of symptoms identifiable in the patient.
Since the levels of CCR2 expressing cells, levels of CCR2 expression or levels of one or more of CCR2^{hi} cells are diagnostically relevant, determining such levels in a sample obtained from a subject may influence treatment selection for that subject. Accordingly, in certain embodiments the invention provides a method of selecting a suitable treatment for multiple sclerosis comprising determining:
a) the levels of the one or more of chemokine receptor CCR2 expressing cells
b) levels of expression of f CCR2; and/or
c) levels of cells with high expression of CCR2
in a sample obtained from a subject, wherein high levels of CCR2 expressing cells, high levels of expression of CCR2 or high levels of cells with high expression of CCR2 or increased levels of CCR2 expressing cells compared to control, increased levels of expression of CCR2 compared to a control or increased levels of cells with high expression of CCR2 compared to a control, result in selection of a treatment of the multiple sclerosis according to the invention. The cells may be lymphocytes, in particular T-lymphocytes. The cells are CCR2 expressing cells, such as T-cells, in specific embodiments.

The various embodiments of the invention will now be described in more detail by reference to the following examples:

### DESCRIPTION OF THE FIGURES

FIG. 1a, 1b & 1c - the binding of biotinylized MCP-1 by CD4+, CD8+ T-cells and CD14+ monocytes respectively, obtained from peripheral blood of a healthy donor.
FIG. 2a - binding of MCP-1 to monocytes (dashed line) in peripheral blood taken from IBD patients. The graph represents a summary of four tests.
FIG. 2b - binding of CCR2-antibody to monocytes (line) in peripheral blood taken from IBD patients. The graph represents a summary of four tests.
FIG. 3 - The plastic house and top showing the distribution plate (2) and safety filter units (3 and 4).
FIG. 4 - The overall leukapheresis system
FIG. 5 - The pump with air detector and optical detector (4).
FIG. 6a - Results of in vitro depletion tests performed on the bMCP-1 coupled matrix showing ability to eliminate CCR2-expressing cells from blood from three healthy donors.
FIG. 6b - Results of in vitro depletion tests performed on the biotinylated RANTES coupled matrix showing ability to eliminate chemokine receptor-expressing cells from peripheral blood of a healthy donor.
FIG. 6c - Results of in vitro depletion tests performed on the biotinylated MIP-3a coupled matrix showing ability to eliminate CCR6-expressing lymphocytes from blood from three healthy donors.
FIG. 7 - Sequence and biotinylation, via a spacer group, of mature protein MCP-1 derivative containing Gln to pyroGlu modification
FIG. 8 - Sequence and biotinylation, via a spacer group, of mature protein MCP-1 derivative containing Gln to pyroGlu modification and Met to Norleu substitution
FIG. 9 - Sequence and biotinylation, via a spacer group, of truncated MCP-1 derivative containing Met to Norleu substitution
FIG. 10 - Alignment of MCP-1 and MCP-5 amino acid sequences
FIG. 11 - Sequence and biotinylation, via a spacer group, of (C-terminal) truncated MCP-5 derivative containing Ile to Lys modification
FIG. 12 - Sequence and biotinylation, of RANTES derivative
FIG. 13 - example of gating criteria for CCR2 expressing monocytes
FIG. 14a - Frequency of CCR2 positive T cells. Bars represent mean and SEM of T cells that express CCR2 in 2 patients and 20 healthy controls.
FIG. 14b - Frequency of CCR6 positive T cells. Bars represent mean and SEM of T cells that express CCR6 in 5 patients and 20 healthy controls. The expression of chemokine receptors and specific cell markers were analysed with flow cytometry. The T cells were characterized as CD3 positive.
FIG. 15a - Binding of the chemokine bMCP-1 to T cells. Bar represents mean frequency and SEM of MCP-1 binding T cells in 5 patients with MS.
FIG. 15b - Binding of the chemokine bMIP3a to T cells. Bar represents frequency of MIP3a-binding T cells in 1 patient with MS. Blood was incubated with biotinylated chemokine and analysed with flow cytometry. The T cells were characterized as CD3 positive.
FIG. 16a - Depletion of CCR2 expressing T cells with Sepharose Streptavidin-matrix conjugated with bMCP-1.
FIG. 16b - Depletion of CCR6 expressing T cells with Sepharose Streptavidin-matrix conjugated with bMIP3a. Blood cells from a patient with MS were incubated with biotinylated chemokine-Sepharose Streptavidin-matrix. Unbound cells were retrieved by washing the matrix. The cells (After Depletion) were then analysed with flow cytometry and compared with cells that had not been incubated with bchemokine-matrix (Before Depletion).

### DESCRIPTION OF PREFERRED EMBODIMENTS

In secondary progressive MS microglia/ MØ present at border of plaques produce chemokines MCP-1 and CXCL10 responsible for attracting CCR2 and CXCR3 expressing cells including macrophages and astrocytes.

It is shown herein that subjects suffering from MS exhibit increased frequency of chemokine receptor expressing cells in the peripheral blood, in particular CCR2 and CCR6 expressing T lymphocytes, compared to healthy controls. It is also shown herein that the CCR2 cells can be removed using a suitable binding reagent, in particular MCP-1 (in biotinylated form) immobilized on a suitable matrix. Similarly, it is shown herein that (the additional) CCR6-expressing cells can be depleted using a suitable binding reagent, in particular CCL20 (MIP-31), in biotinylated form, immobilized on a suitable matrix.

The CCL5 levels are significantly elevated in cerebrospinal fluid of MS patients with relapsing disease demonstrating that circulating CCL5 is involved in recruiting CCL5 binding cells to the brain. These findings are supported by the enrichment of T cells in cerebrospinal fluid expressing CCR5 and CCR6 suggesting an active accumalation due to a chemokine gradient of CCL5. Therefore eliminating cells normally attracted to the brain by providing an extracorpeal source of CCL5 attached to a column will be useful for the treatment of MS.

### EXAMPLES 1 and 2

### Materials and methods

*Isolation of peripheral blood leukocytes.* Heparinized peripheral blood from healthy blood donors or inflammatory bowel disease (IBD) patients was fixed with 4% paraformaldehyde for 4 minutes, hemolyzed for 15 minutes with a 0.83 % ammonium chloride solution and washed twice in FACS buffer to obtain a suspension of blood leukocytes.

*Chemokines.* The leukocytes were incubated for 30 min in the dark at 4 °C with biotinylated and Alexa647 Fluor® labeled MCP-1 (in concentrations 10 ng/µL and 50 ng/µL). The cells were then washed with FACS-buffer and analyzed by flow cytometry. All chemokines used in the Examples were provided by Almac Sciences Scotland Ltd, Edinburgh, Scotland.

*Flow cytometry assay.* The flow cytometry assay was performed on a two laser FACS Calibur cytometer (BD Immunocytometry systems, San José, Ca, USA). Ten thousand cells were counted and analysed in each sample. For data analyses, Cell Quest Pro software from Becton Dickinson was used.

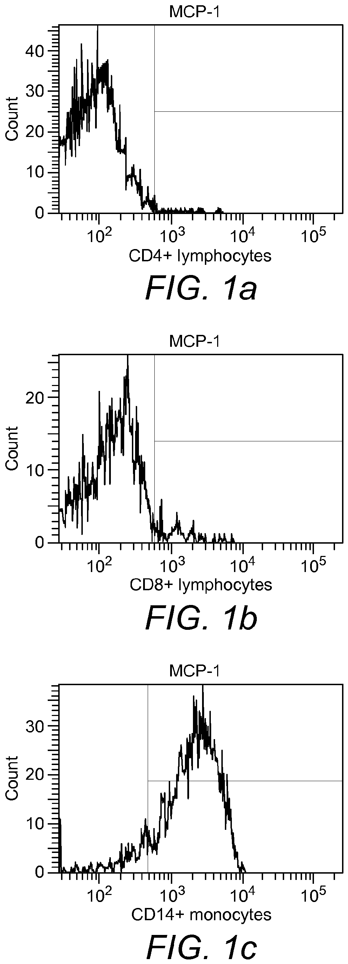

**EXAMPLE 1-** *Binding of monocytes to MCP-1.* In the experiment with biotinylated MCP-1 it was found that about 90% of the monocytes obtained from peripheral blood of healthy donors had bound to the cytokine after 30 min of incubation (Fig. 1a), whereas CD4+ and CD8+ lymphocytes had not bound (Fig. 1b and 1c).

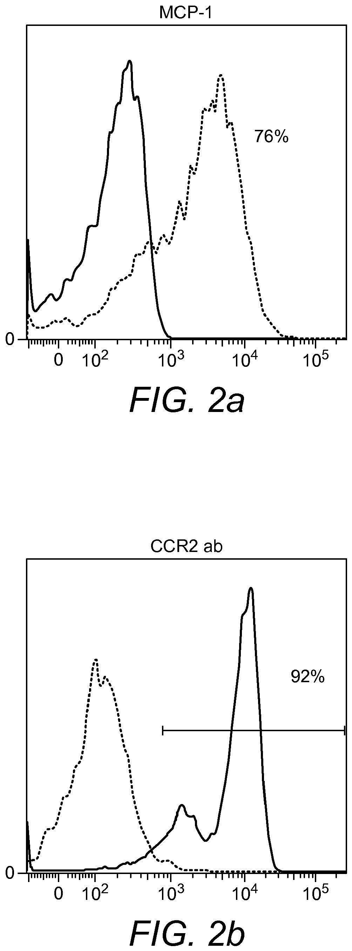

**EXAMPLE 2 -** Monocytes were investigated for their expression of CCR2 (FIG. 2b) and their ability to bind MCP-1 (FIG. 2a). CCR2 expression was noted an all monocytes with the majority of monocytes expressing high levels, using an anti-CCR2 antibody (FIG. 2b). The MCP-1 binding to monocytes shown in FIG. 2a corresponds to the CCR2hi expressing population shown in FIG. 2b. Thus, MCP-1 binds favourably to CCR2hi expressing cells.

### EXAMPLE 3 - Tailored leukapheresis

### COLUMN DESIGN AND PROPERTIES

### Introduction

Apheresis is an established treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. Side effects of leukapheresis treatments are varying from mild events like headache, dizziness, hypotension, palpitation and flush seen in 0.1 to 5% of treated patients.

### The column

The column is intended to be used as a leukapheresis treatment for multiple sclerosis. It will specifically remove CCR2, CCR6, CCR3, CCR5, CCR1, CXCR3 and/or CCR9 -expressing leukocytes, in particular monocytes, through the use of a binding reagent, more specifically an MCP-1, MCP-2, MCP-3, MCP-4, MCP-5, MIP-3alpha, MIG (CXCL9), IP10 (CXCL10), CXCL11 (I-TAC), CCL25 and RANTES containing resin, exploiting the CCR2, CCR6, CCR3, CCR5, CCR1, CXCR3 and/or CCR9 -chemokine interaction. The column consists of three combined components, the plastic house, the streptavidin (SA) Sepharose™ BigBeads matrix and one or more of biotinylated MCP-1, MCP-2, MCP-3, MCP-4, MCP-5, MIP-3alpha, MIG (CXCL9), IP10 (CXCL10), CXCL11 (I-TAC), CCL25 and RANTES bound to the matrix. The treatment is conducted using the same techniques as a standard apheresis procedure.

### The plastic house (FIG. 3)

The plastic house, designed to keep a continuous blood flow through the matrix, consists of a transparent body and red-coloured top. The top has a distribution plate (2) at the inflow site (1) to spread the blood evenly over the entire matrix area. The plate is the first safety barrier preventing larger particles flowing through the column and into the patient. Safety filter units (3 and 4) are placed at the inflow (1) and outflow (5) sites of the plastic housing. The safety filter unit contains three filters designed to be a robust barrier and stop all particles larger than blood cells passing through the column. The plastic housing design is shown in Figure 3. The design with safety filters (3 and 4) at both ends of the column device will minimize the risk of leakage of particles into the patient, including in the event that the device is placed up side down with the blood flow in the opposite direction to that anticipated.

### Streptavidin Sepharose™ BigBeads

The second component in the device is the affinity matrix called streptavidin Sepharose™ BigBeads (Sepharose™ GE Healthcare, Sweden). Sepharose™ is a cross linked, beaded-form of agarose, which is a polysaccharide extracted from seaweed. Sepharose™ and agarose are commonly used as column matrices in biomedical affinity techniques. It is chosen for its optimal distribution capacity and can provide a large available area for affinity binding.

### Binding reagent

Coupled to the matrix is the third component of the device, the one or more binding reagents that bind specifically to CCR2, CCR6, CCR3, CCR5, CCR1, CXCR3 and/or CCR9. One or more chemokines such as those selected from the group consisting of MCP-1, MCP-2, MCP-3, MCP-4, MCP-5, MIP-3alpha, MIG (CXCL9), IP10 (CXCL10), CXCL11 (I-TAC), CCL25 and RANTES may be employed. These peptides may be synthetic, engineered versions of the human chemokine, which are truncated and biotinylated, but retain binding activity to the CCR2, CCR6, CCR3, CCR5, CCR1, CXCR3 and/or CCR9 receptor. By biotinylating the engineered chemokine, it is able to bind to the streptavidin molecules in the Sepharose™ matrix. The biotin-streptavidin binding is known be one of the strongest biological interactions with a Kd in the order of 4 x 10⁻¹⁴ M. The calculated ratio of streptavidin:biotin binding sites in the column is 10:1. Therefore, the coupling between the matrix and chemokine will be immediate, minimising the risk of chemokine decoupling from the matrix.

### The apheresis system

To conduct the leukapheresis the following components are needed; the column, tubing system, and a 4008 ADS pump (Fresenius Medical Care).

### The circuit

The system is illustrated in Figure 4. The patient (1) is connected to the extracorporeal circuit via sterile Venflon needles to veins in the right and the left arms. A saline bag (3) is also connected and the saline solution is pumped with an ACD pump (2). Blood is drawn from one arm of the patient through the sterile tubing system by the blood pump (4) and passed through the column (6) and back to the patient. The tubing system is connected to the column via standard dialysis luer-lock couplings. The couplings on the column are colour-coded for correct assembly; red tubing for inflow to the red column top and blue tubing for outflow back to the patient. An air detector (8) is present. Inlet pressure(5) and Pven sensors (7) are employed to monitor the pressure in the circuit.

### The 4008 ADS pump

An apheresis pump, from Fresenius Medical Care, monitors the patient's inflow and outflow, the pressure in the extracorporeal circulation and can discriminate air by a bubble catcher and air detector. A clot catcher filter is placed inside the bubble catcher. The pump also has an optical detector to distinguish between light, e.g. saline solution or air present in the tubing system and dark e.g. blood present in the tubing system.
A schematic diagram of the pump, showing the air detector and optical filter is shown in Figure 5. If the pump system detects air bubbles and optical fluctuations or if extracorporeal pressure values are out of the set range, then the pump stops immediately and a visual/ audible alarm are emitted.

### Legend for FIG. 5:

- 1.: Monitor
- 2.: Holder for waste bag
- 3.: Modules (left to right - Blood pump, ACD pump, Air detector)
- 4.: Reserve places for further modules
- 5.: Absorber holder
- 6.: Drip detector
- 7.: IV pole

### Preparation of the patient

The patient will be administered anticoagulants prior to each treatment session. A sterile saline solution with 5000 IE Heparin will be used for priming the extracorporeal system, thereafter a bolus injection with 4000 IE Heparin will be added into the circuit at the start of each treatment session.

### Leukapheresis time and flow rate

The apheresis system should be operated at a flow rate of 30-60 mL/min. A treatment is finalised after 1800mL of blood has been circulated.

### Storage conditions

The column devices should be stored between 1 and 25°C avoiding freezing and more elevated temperatures. Stability data > 3 months indicate no difference in functionality over time or by temperature (room temperature and refrigerated). The columns will be kept in refrigerated conditions until use. Mechanical damage as those resulting from violent vibrations and trauma should be avoided. Column stored outside of these recommendations should not be used.

### Transport conditions

The column devices will be transported under refrigerated condition, avoiding freezing and more elevated temperatures. Mechanical damage such as those resulting from violent vibrations and trauma should be avoided.

### In-vitro depletion of target cell populations - MCP-1

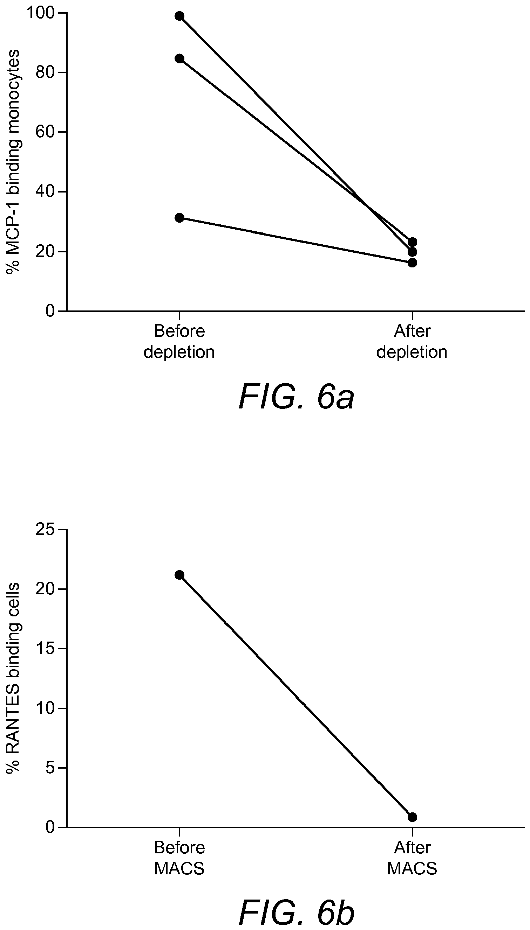

To investigate the ability to eliminate CCR2-expressing cells, in vitro tests have been performed on the bMCP-1 coupled matrix. Blood was collected from blood donors and passed through the column device containing bMCP-1 coupled matrix. Blood samples were taken before and after column passage and analyzed by flow cytometry (FACS) for the depletion of CCR2-expressing cells.
The results demonstrate significant depletion of the target population CCR2-expressing monocytes post matrix perfusion. Depletion tests were performed on blood from three healthy donors. The results are shown in Figure 6a.
In conclusion, the in-vitro results demonstrate a specific reduction of up to 80% of the CCR2-expressing cells by the column. Notably, individuals with fewer CCR2 expressing cells initially achieved lower depletion. The remaining levels of monocytes were around 20-30% in each case, irrespective of the starting point. Non-CCR2-expressing cells remained unaffected (data not shown).

### In-vitro depletion of target cell populations - RANTES

To investigate the ability to eliminate CCR1, 3 and 5-expressing cells, in vitro tests have been performed on the biotinylated RANTES coupled matrix. Blood was collected from blood donors and passed through the column device containing biotinylated RANTES coupled matrix. Blood samples were taken before and after column passage and analyzed by flow cytometry (FACS) for the depletion of CCR1, 3 and 5-expressing cells.
The results demonstrate significant depletion of the target population chemokine receptor-expressing cells post matrix perfusion. Depletion tests were performed on blood from a healthy donor. The results are shown in Figure 6b.

The in-vitro results demonstrate a specific reduction of around 20% of the chemokine receptor-expressing cells by the column. Non-CCR1, 3 or 5-expressing cells remained unaffected (data not shown).

### In-vitro depletion of target cell populations - MIP-3alpha

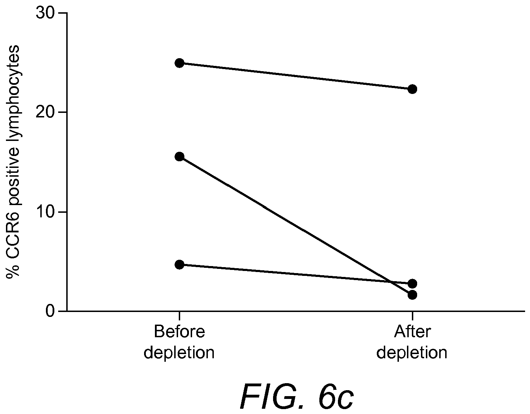

To investigate the ability to eliminate CCR6-expressing cells, in vitro tests have been performed on the biotinylated MIP-3a coupled matrix. Blood was collected from blood donors and passed through the column device containing biotinylated MIP-3a coupled matrix. Blood samples were taken before and after column passage and analyzed by flow cytometry (FACS) for the depletion of CCR6-expressing cells.
The results demonstrate significant depletion of the target population CCR6-expressing lymphocytes post matrix perfusion. Depletion tests were performed on blood from three healthy donors. The results are shown in Figure 6c.
The in-vitro results demonstrate a specific reduction of up to around 15% of the CCR6-expressing cells by the column. Non-CCR6-expressing cells remained unaffected (data not shown).

The RANTES molecule was synthesized by Almac. The amino acid sequence of the biotinylated RANTES molecule is set forth as SEQ ID NO: 17:

This molecule has the naturally occurring methionine at postion 67 replaced with lysine to facilitate biotinylation at position 67.

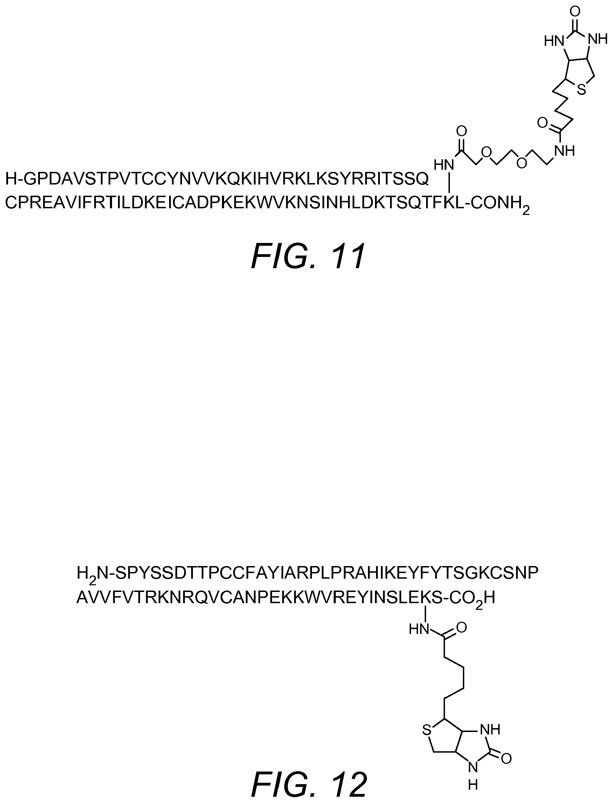

The side-chain of Lys 67 was directly biotinylated to given the protein primary structure shown in figure 12. The protein was folded and disulphide bonds formed between the first and third cysteine in the sequence and between the 2nd and 4th cysteines.

### EXAMPLE 4 - MCP1 DERIVATIVES

MCP-1 has been produced with residue 75 as the site of biotinylation on the chemokine (numbering based upon the mature protein having the amino acid sequence of SEQ ID NO: 2). Biotinylation permits immobilization of MCP-1 on a solid support (via a biotin-avidin interaction). The basic amino acid sequence of MCP-1, including a 23 amino acid leader sequence is set forth as SEQ ID NO: 1,

The amino acid sequence of the mature protein is set forth as SEQ ID NO: 2, X = Met or Nleu
The inventors have determined that chemokines may display improved binding properties where the chemokine is biotinylated via a spacer group. The spacer may prevent the biotin group from impacting on the binding affinity of the chemokine.

Thus, MCP-1 derivatised at the ε-amino side chain functionality of Lys75 with PEG-Biotin (TFA salt) will be synthesised. The PEG spacer will be 3,6, - dioxoaminooctanoic acid. The Gln at the N-terminus of the proteins is subject to pyroGlu formation under physiological conditions. Thus the first glutamine (Gln1) of the sequence will be substituted with pyroglutamine. The molecule will be synthesised as a C-terminal amide (via synthesis on an amide linker). The molecule is shown schematically in Figure 7.

A biotinMCP-1 Met to Nleu analogue will also be synthesised. The single methionine within the sequence will be altered to Norleucine, to mitigate against oxidation of this residue during the chain assembly and improve stability of the final product. This molecule is shown schematically in Figure 8 and in SEQ ID NO: 2.

Once synthesised, the activity of the various biotinMCP-1 derivatives will be determined in cell-based assays. In particular, agonist and antagonist properties will be determined in aequorin functional cell-based assay on human CCR2 receptor.

### EXAMPLE 5 - SYNTHESIS OF A CCR2 ANTAGONIST BIOTINMCP-1 WHICH BINDS TO THE RECEPTOR WITHOUT ACTIVATION

Antagonist Activity (J-H Gong and I. Clark-Lewis, J. Exp. Med., 1995, 181, 63) has been shown for an MCP-1 derivative truncated at the N-terminus. In particular, deletion of residues 1-8, results in binding to CCR2 with Kd 8.3 nM. This protein was unable to cause chemotaxis of CCR2 positive cells (inhibition of chemotaxis IC50 20nM)

The amino acid sequence of the truncated version is set forth as SED ID NO: 3:

A derivative of this truncated version will be synthesised comprising residues 9 to 76 of the mature protein (MCP-1 9-76) with Met64 to Nleu substitution and derivatised at the ε-amino side chain functionality of Lys75 with PEG-Biotin (TFA salt). This molecule is shown schematically in Figure 9. The PEG spacer will be 3,6, - dioxoaminooctanoic acid.

Once synthesised, the activity of the various biotinMCP-1 derivatives will be determined in cell-based assays. In particular, agonist and antagonist properties will be determined in aequorin functional cell-based assay on human CCR2 receptor.

### EXAMPLE 6 - DEMONSTRATE REMOVAL OF CCR2 EXPRESSING CELLS USING AN ALTERNATIVE CHEMOKINE LIGAND TO MCP-1 - Reference Example

CCR2 also binds chemokines MCP-2, MCP-3, MCP-4, MCP-5, and HCC-4 in addition to MCP-1. MCP-5 only binds CCR2 and should be selective in its removal of CCR2 expressing cells. MCP5 is a mouse chemokine shown to chemotact human CCR2 cells with EC50 < 3 nM.

The full length amino acid sequence, including the signal peptide, is set forth as SEQ ID NO: 4

The amino acid sequence of N-terminal processed MCP-5 chemokine is 82 amino acids long and is set forth as SEQ ID NO: 5

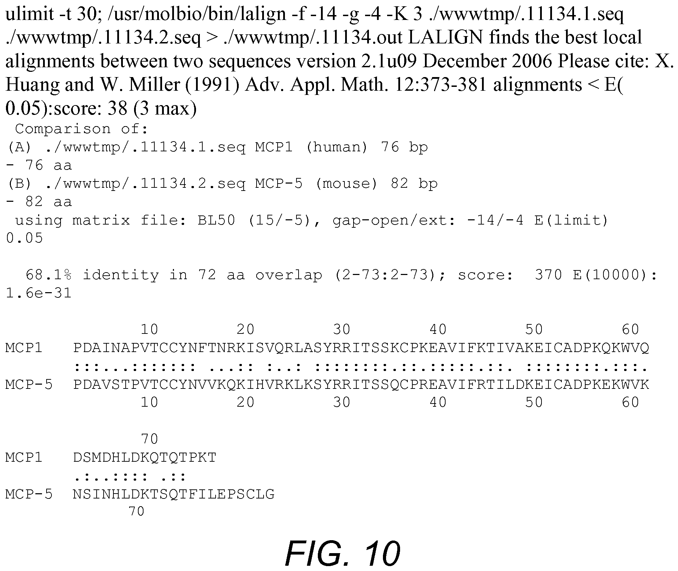

An amino acid sequence alignment suggests that MCP-5 has a C-terminal extension when compared to the amino acid sequence of MCP-1. The results of this alignment are shown in figure 10. On this basis a C-terminal truncated version of MCP-5 will be synthesised. This truncated version will comprise MCP-5 residues 1-76, set forth as SEQ ID NO: 6:

In the truncated version, Ile75 to be substituted with Lys, set forth as SEQ ID NO: 7:

Following substitution, the substituted version will be biotinylated at position 75, a lysine or other suitable residue such as ornithine or diaminopropanoic acid via A PEG spacer (3,6, - dioxoaminooctanoic acid). The protein will be synthesised on an amide linker to yield a C-terminal amide derivative. This molecule is shown schematically in Figure 11.

Once synthesised, the activity of the various biotinMCP-5 derivatives will be determined in cell-based assays. In particular, agonist and antagonist properties will be determined in aequorin functional cell-based assay on human CCR2 receptor.

### EXAMPLES 7 to 14

### General Protocols for Chemokine Synthesis

### Assembly:

Chemical synthesis of chemokines was performed using standard Fmoc solid phase peptides synthesis (SPPS) techniques on an ABI 433 peptide synthesiser. DIC (0.5 M in DMF) and OxymaPure (0.5 M in DMF) were used for activation, acetic anhydride (0.5 M in DMF) for capping, and 20 % piperidine in DMF for Fmoc deprotection. Rink Amide resin was utilised for the generation of C-terminal amide chemokines and Wang resin for C-terminal acid chemokines. After assembly, the resin was washed with DMF and DCM and then dried *in vacuo.*

### Removal of Dde Protection:

The Dde protecting group was removed by treatment of resin with a solution of 2.5% hydrazine in DMF (200ml) over a 2 hour period. The resin was then washed with DMF.

### Labelling steps:

### 1. Couple Fmoc-8-amino-3,6-dioctanoic acid (PEG)

Resin was swollen in DMF and then a solution of Fmoc-8-amino-3,6-dioctanoic acid (0.38g, 1mmol), DIC solution (2ml, 0.5 M in DMF) and OxymaPure solution (2ml, 0.5 M in DMF) was added. The mixture was sonicated for 3 hours and then washed with DMF.

### 2. Capping

The resin was capped with acetic anhydride solution (0.5 M in DMF, 10ml) for 5 minutes and then washed with DMF.

### 3. Fmoc deprotection

Fmoc deprotection was carried out by treatment with 20% piperidine in DMF solution (2 x 50ml) for 15 minutes each. The resin was washed with DMF.

### 4. Couple Biotin-OSu

A solution of Biotin-OSu (341 mg, 1mmol) and DIPEA (348µl) in DMF (10ml) was added to the resin and the mixture was sonicated for 3 hours. The resin was washed thoroughly with DMF and DCM then dried *in vacuo.*

### Cleavage:

Dry resin was treated with TFA (10 ml) containing a scavenger cocktail consisting of TIS (500 µl), thioanisole (500 µl), water (500 µl), DMS (500 µl), EDT (250 µl), NH₄I (500 µg) and phenol (500 µg) and the mixture was stirred at room temperature for 5 hours. The solution was filtered into cold ether and the resin rinsed with TFA. The precipitated peptide was centrifuged, washed with ether, centrifuged and lyophilised.

### Purification Protocol:

The crude peptide was purified by reverse phase HPLC (RP-HPLC) using a Jupiter C18, 250 x 21 mm column, 9 ml/min, eluting with an optimised gradient [Buffer A: water containing 0.1 % TFA, Buffer B: acetonitrile containing 0.1 % TFA].

### Folding Protocol:

Pure peptide (10mg) was dissolved into 6M GnHCl (16 ml) and then rapidly diluted to 2M GnHCl concentration by the addition of 50mM TRIS pH 8.5 (84 ml) containing 0.3mM GSSG and 3mM GSH. The mixture was stirred at room temperature for 24 hours and then analysed by RP-HPLC (Jupiter C18, 250 x 4.6 mm column, 10-60% B over 30 minutes. Purification by RP-HPLC using an optimised gradient afforded the desired product.

### EXAMPLE 7 - biotinMCP-1 (CCL2)

Target Molecule: MCP-1 derivatised at the ε-amino side chain functionality of Lys(75) with PEG-Biotin (TFA salt)

Modifications: Human MCP-1 corresponding to residues 1-76, is initially expressed as 99 amino acids comprising the chemokine fold, and a 23 amino acid signal peptide which is cleaved off. The Gln at the N-terminus of the protein is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 75 was modified through biotinylation on the resin. A PEG spacer was incorporated between the ε-amino functionality and the biotin.

The linear amino acid sequence (SEQ ID NO: 8) is shown, prior to attachment of the PEG spacer and biotin molecules at amino acid 75 (K): X = pyroGlu

The engineered MCP-1 sequence was assembled on a solid support (Rink Amide resin), using Fmoc protocols for solid-phase peptide synthesis as described in the general protocols section: X1 = pyroGlu
X75 = K(ivDde)

FmocLys(ivDde)-OH was incorporated as residue 75 to facilitate site-specific labelling at this position of the protein (SEQ ID NO: 9). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 10): X1 = pyroGlu
X75 = an amino acid residue that can be biotinylated, such as lysine, ornithine or diaminopropionic acid and optionally is biotinylated, optionally via a spacer molecule such as PEG, e.g. K(PEG-Biotin).

Electrospray ionisation with tandem mass spectrometry (ESI-TOF-MS) data of purified folded biotinMCP-1: obtained = 9032.8 Da; expected 9034.4 Da.

### Functional Assay Data:

biotinMCP-1 was tested for agonist activity in an Aequorin assay against hCCR2b, (Euroscreen) and an EC50 value of 9.6nM was reported. c.f. EC50 for recombinant native MCP-1 is 3.1 nM.

### Example 8 - biotinMCP-2 (CCL8) - Reference Example

Target Molecule: MCP-2 derivatised at the ε-amino side chain functionality of Lys(75) with PEG-Biotin (TFA salt)

Modifications: Human MCP-2 corresponding to residues 1-76, is initially expressed as 99 amino acids comprising the chemokine fold, and a 23 amino acid signal peptide which is cleaved off. The Gln at the N-terminus of the protein is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 75 was modified through biotinylation on the resin. A PEG spacer was incorporated between the ε-amino functionality and the biotin.

The linear amino acid sequence (SEQ ID NO: 11) is shown, prior to attachment of the PEG spacer and biotin molecules at amino acid 75 (K): X = pyroGlu

The engineered MCP-2 sequence was assembled on a solid support (Rink Amide resin), using Fmoc protocols for solid-phase peptide synthesis as described in the general protocols section: X1 = pyroGlu
X75 = K(ivDde)

FmocLys(ivDde)-OH was incorporated as residue 75 to facilitate site-specific labelling at this position of the protein (SEQ ID NO: 12). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 13): X1 = pyroGlu
X75 = an amino acid residue that can be biotinylated, such as lysine, ornithine or diaminopropionic acid and optionally is biotinylated, optionally via a spacer molecule such as PEG, e.g. K(PEG-Biotin).

Electrospray ionisation with tandem mass spectrometry (ESI-TOF-MS) data of purified folded biotinMCP-2: obtained = 9263.6 Da; expected 9263.8 Da.

### Functional Assay Data:

biotinMCP-2 was tested for activity in an Aequorin assay against hCCR2b, (Euroscreen) and was shown to be a partial agonist with an EC50 value of 50.9nM. c.f. EC50 for recombinant native MCP-2 is 23.5nM (partial agonist).

### EXAMPLE 9 - biotinEotaxin (CCL11) - Reference Example

Target Molecule: Eotaxin derivatised at the ε-amino side chain functionality of Lys(73) with PEG-Biotin (TFA salt)

Modifications: Human eotaxin corresponding to residues 1-74, is initially expressed as 97 amino acids comprising the chemokine fold, and a 23 amino acid signal peptide which is cleaved off. The naturally occurring lysine at position 73 was modified through biotinylation on the resin. A PEG spacer was incorporated between the ε-amino functionality and the biotin.

The linear amino acid sequence (SEQ ID NO: 14) is shown, prior to attachment of the PEG spacer and biotin molecules at amino acid 73 (K): X is an amino acid residue that can be biotinylated, such as lysine, ornithine or diaminopropionic acid and optionally is biotinylated, optionally via a spacer molecule such as PEG, e.g. K(PEG-Biotin).

The engineered eotaxin sequence was assembled on a solid support (Rink Amide resin), using Fmoc protocols for solid-phase peptide synthesis as described in the general protocols section: X is K(ivDde)

FmocLys(ivDde)-OH was incorporated as residue 73 to facilitate site-specific labelling at this position of the protein (SEQ ID NO: 15). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 16): X is K(PEG-Biotin)

Electrospray ionisation with tandem mass spectrometry (ESi-TOF-MS) data of purified folded biotinEotaxin: obtained = 8731.3 Da; expected 8731.3 Da.

### Functional Assay Data:

biotinEotaxin was tested for activity in an Aequorin assay against hCCR3, (Euroscreen) and was shown to be an antagonist with an EC50 value of 211.8nM. c.f. EC50 for recombinant native eotaxin is 10.7nM (agonist).

### Example 10 - biotinRANTES (CCL5) - Reference Example

Target Molecule: RANTES derivatised at the ε-amino side chain functionality of Lys(67) with Biotin (TFA salt)

Modifications: Human RANTES corresponding to residues 1-68, is initially expressed as 91 amino acids comprising the chemokine fold, and a 23 amino acid signal peptide which is cleaved off. The single methionine (Met67) within the sequence was mutated to lysine, to mitigate against oxidation of this residue during the chain assembly, which was observed during the synthesis of the natural sequence derivative. This Met to Lys substitution provided a lysine at position 67 which was modified through biotinylation on the resin.

The linear amino acid sequence (SEQ ID NO: 17) is shown, prior to attachment of the biotin molecule at amino acid 67 (K):

The engineered RANTES sequence was assembled on a solid support (Wang resin), using Fmoc protocols for solid-phase peptide synthesis as described in the general protocols section: X is K(ivDde)

FmocLys(ivDde)-OH was incorporated as residue 67 to facilitate site-specific labelling at this position of the protein (SEQ ID NO: 18). Subsequent removal of the ivDde protecting group, followed by coupling of the Biotin, was carried out as described in the general protocol section. Cleavage, purification and folding protocols were carried out as described to furnish the desired active chemokine (SEQ ID NO: 19). X is an amino acid residue that can be biotinylated, such as lysine, ornithine or diaminopropionic acid and optionally is biotinylated, optionally via a spacer molecule such as PEG (e.g. K(Biotin))

Electrospray ionisation with tandem mass spectrometry (ESI-TOF-MS) data of purified folded biotinRANTES: obtained = 8068.9 Da; expected 8070.2 Da.

### Functional Assay Data:

BiotinRANTES was tested for agonist activity in an Aequorin assay against hCCR5, (Euroscreen) and an EC50 value of 0.5nM was reported.

### Example 11 - biotinMIP-3α (CCL20) - Reference Example

Target Molecule: MIP-3α derivatised at the ε-amino side chain functionality of Lys(68) with PEG-Biotin (TFA salt)

Modifications: Human MIP-3α corresponding to residues 1-70, is initially expressed as 96 amino acids comprising the chemokine fold, and a 26 amino acid signal peptide which is cleaved off. The naturally occurring lysine at position 68 was modified through biotinylation on the resin. A PEG spacer was incorporated between the ε-amino functionality and the biotin.

The linear amino acid sequence (SEQ ID NO: 20) is shown, prior to attachment of the PEG spacer and biotin molecules at amino acid 68 (K):

The engineered MIP-3α sequence was assembled on a solid support (Wang resin), using Fmoc protocols for solid-phase peptide synthesis as described in the general protocols section: X= K(ivDde)

FmocLys(ivDde)-OH was incorporated as residue 68 to facilitate site-specific labelling at this position of the protein (SEQ ID NO: 21). Subsequent removal of the ivDde protecting roup, followed by coupling of the PEG spacer and Biotin, was carried out as described in the general protocol section. Cleavage, purification and folding protocols were carried out as described to furnish the desired active chemokine (SEQ ID NO: 22). X is an amino acid residue that can be biotinylated, such as lysine, ornithine or diaminopropionic acid and optionally is biotinylated, optionally via a spacer molecule such as PEG, in particular K(PEG-Biotin)

Electrospray ionisation with tandem mass spectrometry (ESI-TOF-MS) data of purified folded biotinMip-3α: obtained = 8396.4 Da; expected 8397.0 Da.

### Functional Assay Data:

BiotinMIP-3α was tested for agonist activity in an Aequorin assay against hCCR6, (Euroscreen) and an EC50 value of 1.6nM was reported. c.f. EC50 for recombinant native MIP-3α is 1.0nM.

### Example 12 - biotinTECK (CCL25) - Reference Example

Target Molecule: TECK (Met to Nleu substitution) derivatised at the ε-amino side chain functionality of Lys72 with PEG-Biotin (TFA salt)

Modifications: Truncated form of human TECK corresponding to residues 1-74 of the mature protein, which encompasses the sequence corresponding to the chemokine fold. The full length mature protein is 127 amino acids (the signal peptide is 23 amino acids in a 150 amino acid immature protein). The single methionine within the sequence was altered to Norleucine, to mitigate against oxidation of this residue during the chain assembly, which was observed during the synthesis of the natural sequence derivative. The Gln at the N-terminus of the proteins is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 72 was modified through biotinylation on the resin. A PEG spacer was incorporated between the ε-amino functionality and the biotin.

The linear amino acid sequence (SEQ ID NO: 23) is shown, prior to attachment of the PEG spacer and biotin molecules at amino acid 72 (K): X1 = pyroGlu or Gln
X64 = Norleucine

The engineered TECK sequence was assembled on a solid support (Wang resin), using Fmoc protocols for solid-phase peptide synthesis as described in the general protocols section: X1 = pyroGlu or Gln
X64 = Norleucine
X72 = K(Dde)
NPKSREVQRANleKLLDARNK(ivDde)VF-RESIN

FmocLys(ivDde)-OH was incorporated as residue 72 to facilitate site-specific labelling at this position of the protein (SEQ ID NO: 24). Subsequent removal of the ivDde protecting group, followed by coupling of the PEG spacer and Biotin, was carried out as described in the general protocol section. Cleavage, purification and folding protocols were carried out as described to furnish the desired active chemokine.

The final active chemokine thus has the following sequence (SEQ ID NO: 25): X1 = pyroGlu or Gln
X64 = norleucine
X72 = an amino acid residue that can be biotinylated, such as lysine, ornithine or diaminopropionic acid and optionally is biotinylated, optionally via a spacer molecule such as PEG, such as K(PEG-Biotin)

Electrospray ionisation with tandem mass spectrometry (ESI-TOF-MS) data of purified folded biotinTECK(Met to Nleu substitution): obtained = 8958.5 Da; expected 8959.6 Da.

### Functional Assay Data:

biotinTECK(Met to Nleu substitution) was tested for agonist activity in an Aequorin assay against hCCR9, (Euroscreen) and an EC50 value of 63.6nM was reported. c.f. EC50 for recombinant native TECK is 67.9nM.

### Example 13 - biotinITAC (CXCL11) - Reference Example

Target Molecule: ITAC derivatised with Biotin at the ε-amino side chain functionality of an additional Lysine inserted at the C-terminus after a PEG spacer (TFA salt)

Modifications: Human ITAC corresponding to residues 1-73, is initially expressed as 94 amino acids comprising the chemokine fold, and a 21 amino acid signal peptide which is cleaved off. A PEG spacer and an additional lysine were inserted at the C-terminus, and modified through biotinylation on the resin. The PEG spacer was incorporated at the C-terminus between the protein and the additional lysine.

The linear amino acid sequence (SEQ ID NO: 26) is shown, prior to attachment of the PEG spacer, additional lysine and biotin molecules:

The engineered ITAC sequence was assembled on a solid support (Wang resin), using Fmoc protocols for solid-phase peptide synthesis as described in the general protocols section: X is PEG-K(ivDde)

Fmoc-12-amino-4,7,10-trioxadodecanoic acid followed by FmocLys(ivDde)-OH were incorporated at the C-terminus to facilitate site-specific labelling with biotin at the ε-amino side chain functionality of the additional Lys (SEQ ID NO: 27). Subsequent removal of the ivDde protecting group, followed by coupling of the Biotin, was carried out as described in the general protocol section. Cleavage, purification and folding protocols were carried out as described to furnish the desired active chemokine (SEQ ID NO: 28). X is an amino acid residue that can be biotinylated, such as lysine, ornithine or diaminopropionic acid and optionally is biotinylated, optionally via a spacer molecule such as PEG, e.g. K(PEG-Biotin) and may be attached via a spacer molecule, e.g.PEG-K(Biotin)

Electrospray ionisation with tandem mass spectrometry (ESI-TOF-MS) data of purified folded biotinITAC: obtained = 8866.5 Da; expected 8860.6 Da.

### Functional Assay Data:

biotinITAC was tested for agonist activity in an Aequorin assay against hCXCR3, (Euroscreen) and an EC50 value of 15.7nM was reported. c.f. EC50 for recombinant native ITAC is 0.7nM.

### EXAMPLE 14 - biotinIP-10 (CXCL10) - Reference Example

Target Molecule: IP-10 derivatised with Biotin at the side chain functionality of an additional Lysine inserted at the C-terminus after a PEG spacer (TFA salt)

Modifications: Human IP-10 corresponding to residues 1-77, is initially expressed as 98 amino acids comprising the chemokine fold, and a 21 amino acid signal peptide which is cleaved off. A PEG spacer and an additional lysine were inserted at the C-terminus, and modified through biotinylation on the resin. The PEG spacer was incorporated at the C-terminus between the protein and the additional lysine.

The linear amino acid sequence (SEQ ID NO: 29) is shown, prior to attachment of the PEG spacer, additional lysine and biotin molecules:

The engineered IP-10 sequence was assembled on a solid support (Wang resin), using Fmoc protocols for solid-phase peptide synthesis as described in the general protocols section: X is K(ivDde), optionally attached via a spacer such as PEG, e.g. -PEG-K(ivDde)

Fmoc-8-amino-3,6-dioctanoic acid followed by FmocLys(ivDde)-OH were incorporated at the C-terminus to facilitate site-specific labelling with biotin at the ε-amino side chain functionality of the additional Lys (SEQ ID NO: 30). Subsequent removal of the ivDde protecting group, followed by coupling of the Biotin, was carried out as described in the general protocol section. Cleavage, purification and folding protocols were carried out as described to furnish the desired active chemokine. The final active chemokine thus has the following sequence (SEQ ID NO: 31): X is an amino acid residue that can be biotinylated, such as lysine, ornithine or diaminopropionic acid and optionally is biotinylated, optionally via a spacer molecule such as PEG, e.g. K(PEG-Biotin) and may be attached via a spacer molecule, e.g.PEG-K(Biotin) Electrospray ionisation with tandem mass spectrometry (ESI-TOF-MS) data of purified folded biotinIP-10: obtained = 9141.0 Da; expected 9141.9 Da.

### Functional Assay Data:

BiotinIP-10 was tested for agonist activity in an Aequorin assay against hCXCR3, (Euroscreen) and an EC50 value of 8.7nM was reported. c.f. EC50 for recombinant native IP-10 is 4.4nM.

### EXAMPLE 15 - MS Diagnosis and Treatment based upon CCR2 and CCR6 expressing T-cells

### Materials and methods

### 1. Flow cytometric analysis of peripheral blood

Peripheral blood from patients with Multiple Sclerosis and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH₄Cl, pH 7.5) for 15 min at 37°C. The cells were washed in PBS with 2% Bovine Growth Serum, incubated with 10% human serum for 15min at room temperature (RT) and stained with antibodies (Table 2) at 4°C for 30min. The cells were analysed with flow cytometry on a FACS Canto flow cytometer with the FACSDiva software (BD Biosciences).

**Table 2. List of antibodies for flow cytometric analysis.**

| **Antibody** | **Fluorophore** | **Supplier** |
|---|---|---|
| CD3 | V450 | BD Biosciences |
| CCR6 | PE | BD Biosciences |
| Streptavidin | PE, APC | Biolegend |
| CCR2 | PerCP Cy5.5 | Biolegend |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4Cl, pH 7.5) for 15 min at 37°C. The cells were washed in PBS with 2% Bovine Growth Serum, incubated with 10% human serum 15min at room temperature (RT) and stained with cell specific antibodies together with biotinylated chemokine (1µM) or the corresponding chemokine receptor antibody at 4°C for 30min (Table 2). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40um nylon filter (BD Falcon™ Cell Strainer). The flow through cells were stained with antibodies (Table 2), analysed with flow cytometry and compared with cells from peripheral blood that had not been incubated with the chemokine-matrix.

### Results and Discussion

### 1. Flow cytometric analysis of peripheral blood

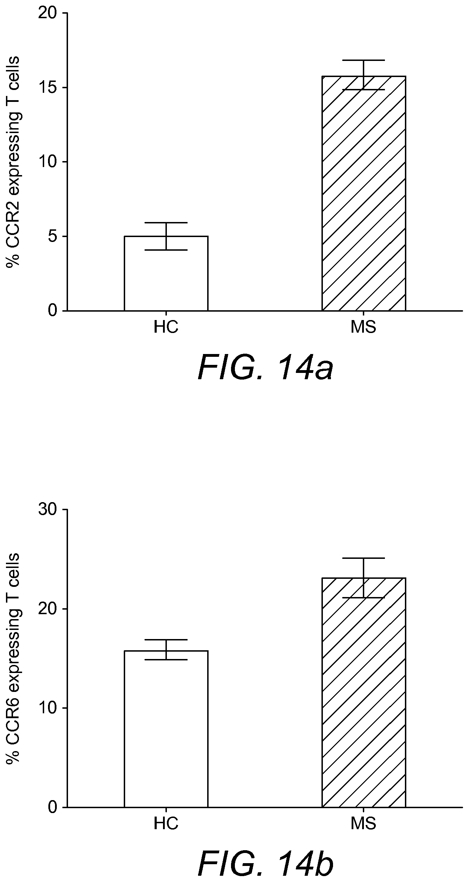

White blood cells from patients with Multiple Sclerosis (MS) were analysed for the expression of chemokine receptors with flow cytometry. The MS patients exhibited an increased frequency of circulating T cells that expressed the chemokine receptor CCR2, 15% compared to approximately 5% in healthy blood (Fig 14a), based upon flow cytometry data and binding by an anti-CCR2 antibody. Furthermore, the patients had an increased frequency of T cells that expressed CCR6 (Fig 14b).

### 2. Chemokine binding test

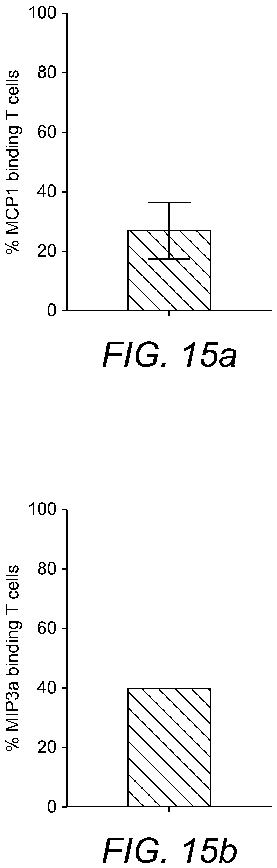

CCR2 binds to the chemokine MCP-1 that mediate migration and infiltration of inflammatory cells to various tissues. The ligand for CCR6 is MIP3a (CCL20) that can mediate migration of T cells into the CNS. Both these receptors are important in the inflammatory process. In accordance with the CCR2 and CCR6 expression, the T cells bound the biotinylated MCP-1 (bMCP-1) (Fig 15a) and bMIP3a (Fig 15b).

### 3. Cell depletion by matrix conjugated with biotinylated chemokine

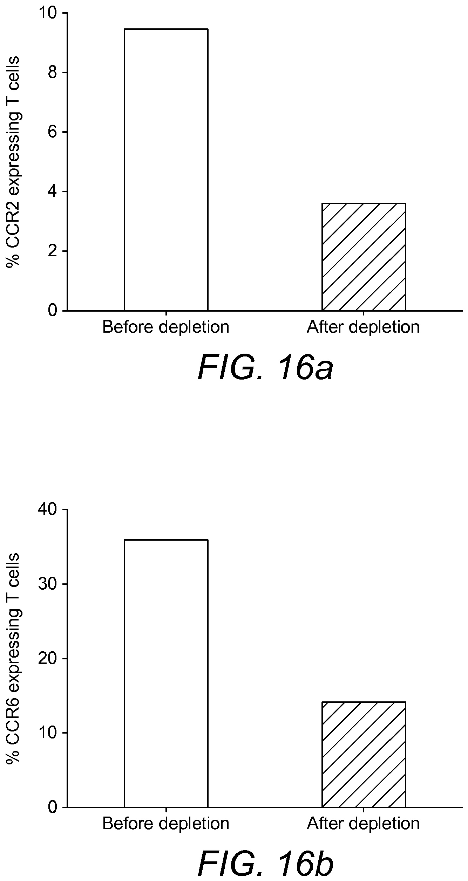

The CCR2 expressing T cells could be efficiently depleted with bMCP-1-conjugated Sepharose Streptavidin Matrix (Fig 16a), and the CCR6 expressing T cells could be depleted with bMIP3a-conjugated Sepharose Streptavidin Matrix (Fig 16b)

We conclude that the frequency of T cells that express CCR2 and CCR6 is enhanced in MS. These T cells can bind the ligands MCP-1 and MIP3a. Furthermore, the majority of the CCR2 and CCR6 expressing T cells can be removed with Sepharose Streptavidin matrix conjugated with the corresponding biotinylated chemokine.

## Claims

1. A binding reagent capable of specifically binding to the chemokine receptor CCR2, for use in the treatment of multiple sclerosis, wherein the binding reagent is immobilized on a solid support contained within an apheresis column, to which is applied peripheral blood from a patient thus removing CCR2 expressing cells from the peripheral blood of the patient, wherein the binding reagent is the MCP-1/CCL2 chemokine.

2. The binding reagent for use according to claim 1 wherein:
(i) the multiple sclerosis is selected from active and stable relapsing-remitting multiple sclerosis, primary progressive, secondary progressive and progressive relapsing multiple sclerosis;
and/or
(ii) the CCR2 expressing cells are selected from monocytes, lymphocytes, neutrophils, macrophages, eosinophils and basophils, optionally
wherein:
the CCR2 expressing cells are monocytes or lymphocytes, optionally T lymphocytes;
and/or
(iii) the patient has been selected for treatment on the basis of detecting an increase in the level of CCR2 expressing cells, levels of expression of CCR2 and/or levels of cells with high expression of CCR2 in a sample obtained from the patient;
and/or
(iv) 20-50% of the patient's blood is applied to the column in a single treatment.

3. A method of selecting a suitable treatment for multiple sclerosis comprising determining
a) the levels of one or more of the chemokine receptor CCR2 expressing cells
b) levels of expression of CCR2; and/or
c) levels of cells with high expression of CCR2
in a sample obtained from a subject, wherein high levels of CCR2 expressing cells, high levels of expression of CCR2 or high levels of cells with high expression of CCR2 or increased levels of CCR2 expressing cells compared to control, increased levels of expression of CCR2 compared to a control or increased levels of cells with high expression of CCR2 compared to a control, result in selection of a treatment as defined in any one of claims 1 to 2 for use in treatment of the multiple sclerosis.

4. The method of claim 3 wherein:
(i) the multiple sclerosis is selected from active and stable relapsing-remitting multiple sclerosis;
and/or
(ii) the sample is a peripheral blood sample or a liquor sample, wherein the cells comprise CCR2 expressing T lymphocytes.

5. The binding reagent for use of claim 1 wherein the binding reagent is a modified MCP-1 chemokine comprising the amino acid sequence set forth as SEQ ID NO: 1, SEQ ID NO: 2 or SEQ ID NO: 3 in which one or more of the following modifications have been made:
a) the glutamine residue at position 1 of SEQ ID NO: 2 has been replaced with pyroglutamine
b) the C terminus is produced as an amide derivative
c) the lysine residue at position 98 of SEQ ID NO: 1 or position 75 of SEQ ID NO:2 or position 67 of SEQ ID NO: 3, which may be replaced by a suitable alternative amino acid such as diaminopropionic acid or ornithine, is biotinylated, optionally via a spacer group, in order to permit immobilization of the chemokine on a solid support
d) the methionine residue at position 87 of SEQ ID NO: 1 or position 64 of SEQ ID NO: 2 or position 56 of SEQ ID NO: 3 has been replaced with norleucine; optionally wherein:
(i) the lysine residue at position 98 of SEQ ID NO: 1 or position 75 of SEQ ID NO: 2 or position 67 of SEQ ID NO: 3 is biotinylated via a polyethylene glycol (PEG) spacer group, in order to permit immobilization of the chemokine on a solid support, optionally wherein the PEG spacer is 3,6-dioxo aminooctanoic acid;
and/or
(ii) the modified MCP-1 chemokine has the structure shown in any one of figures 7 to 9;
and/or
(iii) the modified MCP-1 chemokine is an agonist or an antagonist of CCR2 activity.

6. The binding reagent for use of claim 1 wherein the binding reagent is a modified CCL2 chemokine comprising the amino acid sequence of SEQ ID NO: 10 or SEQ ID NO: 8, optionally wherein:
(i) the residue at position 75 is biotinylated, optionally via a spacer group, in order to permit immobilization of the chemokine on a solid support, optionally wherein the spacer group is a polyethylene glycol (PEG) spacer group and optionally wherein the PEG spacer is 3,6-dioxo aminooctanoic acid;
and/or
(ii) the modified CCL2 chemokine is an agonist or an antagonist of CCR2 activity.

## Patentansprüche

1. Bindungsreagenz, welches zur Verwendung bei der Behandlung von multipler Sklerose an den Chemokinrezeptor CCR2 spezifisch binden kann, wobei das Bindungsreagenz auf einem innerhalb einer Apheresesäule enthaltenen festen Träger immobilisiert wird, auf welche peripheres Blut von einem Patienten aufgetragen wird, wodurch CCR2 exprimierende Zellen aus dem peripheren Blut des Patienten entfernt werden, wobei das Bindungsreagenz das MCP-1 /CCL2-Chemokin ist.

2. Bindungsreagenz zur Verwendung nach Anspruch 1, wobei:
(i) die multiple Sklerose aus aktiver und stabiler schubförmig remittierender multipler Sklerose, primär progressiver, sekundär progressiver und progressiver schubförmig remittierender multipler Sklerose ausgewählt ist;
und/oder
(ii) die CCR2 exprimierenden Zellen aus Monozyten, Lymphozyten, Neutrophilen, Makrophagen, Eosinophilen und Basophilen ausgewählt sind, wahlweise
wobei:
die CCR2 exprimierenden Zellen Monozyten oder Lymphozyten, wahlweise T-Lymphozyten, sind;
und/oder
(iii) der Patient auf Grundlage eines Detektierens einer Erhöhung des Pegels von CCR2 exprimierenden Zellen, des Pegels der Expression von CCR2 und/oder des Pegels von Zellen mit hoher Expression von CCR2 in einer von dem Patienten erhaltenen Probe für Behandlung ausgewählt worden ist; und/oder
(iv) 20-50% des Bluts des Patienten in einer einzigen Behandlung auf die Säule aufgetragen wird.

3. Verfahren zum Auswählen einer geeigneten Behandlung multipler Sklerose umfassend das Bestimmen von
a) dem Pegel einer oder mehrerer der Chemokinrezeptor CCR2 exprimierenden Zellen
b) dem Pegel der Expression von CCR2; und/oder
c) dem Pegel von Zellen mit hoher Expression von CCR2 in einer von einem Individuum erhaltenen Probe, wobei hohe Pegel von CCR2 exprimierenden Zellen, hohe Pegel der Expression von CCR2 oder hohe Pegel von Zellen mit hoher Expression von CCR2 oder erhöhte Pegel von CCR2 exprimierenden Zellen im Vergleich zu einer Kontrolle, erhöhte Pegel der Expression von CCR2 im Vergleich zu einer Kontrolle oder erhöhte Pegel von Zellen mit hoher Expression von CCR2 im Vergleich zu einer Kontrolle, in dem Auswählen einer Behandlung wie in einem der Ansprüche 1 bis 2 angegeben zur Behandlung der multiplen Sklerose resultieren.

4. Verfahren nach Anspruch 3, wobei:
(i) die multiple Sklerose aus aktiver und stabiler schubförmig remittierender multipler Sklerose ausgewählt ist;
und/oder
(ii) die Probe eine periphere Blutprobe oder eine Liquorprobe ist, wobei die Zellen CCR2 exprimierende T-Lymphozyten umfassen.

5. Bindungsreagenz zur Verwendung nach Anspruch 1, wobei das Bindungsreagenz ein modifiziertes MCP-1-Chemokin ist, das die Aminosäuresequenz gemäß SEQ ID NO: 1, SEQ ID NO: 2 oder SEQ ID NO: 3 umfasst, worin eine oder mehrere der folgenden Modifikationen ausgeführt worden sind:
a) der Glutamin-Rest an der Position 1 von SEQ ID NO: 2 ist durch Pyroglutamin ersetzt worden
b) das C-terminale Ende ist als ein Amidderivat hergestellt
c) der Lysin-Rest an der Position 98 von SEQ ID NO: 1 oder an der Position 75 von SEQ ID NO:2 oder an der Position 67 von SEQ ID NO: 3, welcher durch eine geeignete alternative Aminosäure, wie beispielsweise Diaminopropionsäure oder Ornithin, ersetzt sein kann, ist biotinyliert, wahlweise durch eine Spacergruppe, um einer Immobilisierung des Chemokins auf einem festen Träger zu gestatten
d) der Methionin-Rest an der Position 87 von SEQ ID NO: 1 oder an der Position 64 von SEQ ID NO: 2 oder an der Position 56 von SEQ ID NO: 3 ist durch Norleucin ersetzt worden; wahlweise wobei:
(i) der Lysin-Rest an der Position 98 von SEQ ID NO: 1 oder an der Position 75 von SEQ ID NO: 2 oder an der Position 67 von SEQ ID NO: 3 durch eine Polyethylen-Glycol- (PEG)-Spacergruppe biotinyliert ist, um einer Immobilisierung des Chemokins auf einem festen Träger zu gestatten, wahlweise wobei der PEG-Spacer 3,6-Dioxoaminooctansäure ist;
und/oder
(ii) das modifizierte MCP-1-Chemokin die Struktur wie in irgendeiner der Figuren 7 bis 9 gezeigt aufweist;
und/oder
(iii) das modifizierte MCP-1-Chemokin ein Agonist oder ein Antagonist mit CCR2-Aktivität ist.

6. Bindungsreagenz zur Verwendung nach Anspruch 1, wobei das Bindungsreagenz ein modifiziertes CCL2-Chemokin ist, das die Aminosäuresequenz gemäß SEQ ID NO: 10 oder SEQ ID NO: 8 umfasst, wahlweise wobei:
(i) der Rest an der Position 75 biotinyliert ist, wahlweise durch eine Spacergruppe, um einer Immobilisierung des Chemokins auf einem festen Träger zu gestatten, wahlweise wobei die Spacergruppe eine Polyethylen-Glycol- (PEG)-Spacergruppe ist, und wahlweise wobei der PEG-Spacer 3,6-Dioxoaminooctansäure ist;
und/oder
(iii) das modifizierte CCL2-Chemokin ein Agonist oder ein Antagonist mit CCR2-Aktivität ist.

## Revendications

1. Réactif de liaison capable de se lier spécifiquement à un récepteur de chimiokine CCR2 à utiliser dans le traitement de la sclérose en plaques, ledit réactif de liaison étant immobilisé sur un support solide contenu dans une colonne d'aphérèse, à laquelle est appliqué le sang périphérique d'un patient, éliminant ainsi des cellules exprimant CCR2 du sang périphérique du patient, le réactif de liaison étant la chimiokine MCP-1/CCL2.

2. Réactif de liaison pour l'usage selon la revendication 1, dans lequel:
(i) la sclérose en plaques est choisie parmi la sclérose en plaques rémittente-récidivante active et stable, la sclérose en plaques progressive primaire, progressive secondaire et la sclérose en plaques rémittente progressive ;
et / ou
(ii) les cellules exprimant CCR2 sont choisies parmi les monocytes, les lymphocytes, les neutrophiles, les macrophages, les éosinophiles et les basophiles, éventuellement
dans lequel,
les cellules exprimant CCR2 sont des monocytes ou lymphocytes, éventuellement des lymphocytes T ;
et / ou
(iii) le patient a été sélectionné pour le traitement sur la base de la détection d'une augmentation du niveau des cellules exprimant CCR2, des niveaux d'expression de CCR2 et/ou des niveaux de cellules avec une expression élevée de CCR2 dans un échantillon obtenu à partir du patient; et / ou
(iv) 20-50% du sang du patient est appliqué sur la colonne dans un seul traitement.

3. Procédé destiné à sélectionner un traitement approprié de la sclérose en plaques, comprenant la détermination
a) des niveaux d'un ou de plusieurs cellules exprimant le récepteur de chimiokine CCR2
b) des niveau d'expression de CCR2 ; et / ou
c) des niveaux de cellules avec une expression élevée de CCR2 dans un échantillon obtenu à partir d'un sujet, dans lequel des niveaux élevés des cellules exprimant CCR2, des niveaux élevés d'expression de CCR2 ou des niveaux élevés de cellules avec une expression élevée de CCR2 ou des niveaux élevés des cellules exprimant CCR2 par rapport au contrôle, des niveaux élevés d'expression de CCR2 par rapport à un contrôle ou des niveaux élevés des cellules avec une expression élevée de CCR2 par rapport au contrôle entraînent la sélection d'un traitement tel que défini dans l'une quelconque des revendications 1 à 2 pour le traitement de la sclérose en plaques.

4. Procédé selon la revendication 3, dans lequel:
(i) la sclérose en plaques est choisie parmi la sclérose en plaques rémittente-récidivante active et stable ;
et / ou
(ii) l'échantillon est un échantillon de sang périphérique ou un échantillon de liqueur, dans lequel les cellules comprennent des lymphocytes T exprimant CCR2.

5. Réactif de liaison pour l'usage selon la revendication 1, où le réactif de liaison est une chimiokine MCP-1 modifiée comprenant la séquence d'acide aminé telle que SEQ ID NO: 1, SEQ ID NO: 2 ou SEQ ID NO: 3 dans laquelle l'une ou plusieurs des modifications suivantes ont été réalisées :
a) le résidu de lysine à la position 1 de SEQ ID NO: 2 a été remplacé par pyroglutamine
b) l'extrémité C est produite sous la forme d'un dérivé amide
c) le résidu de lysine à la position 98 de SEQ ID NO: 1 ou position 75 de SEQ ID NO: 2 ou la position 67 de SEQ ID NO: 3, qui peut être remplacée par un acide aminé alternatif approprié tel que l'acide diaminopropionique ou l'ornithine, est biotinylée, éventuellement par un groupe d'espaceur, pour permettre l'immobilisation de la chimiokine sur un support solide
d) le résidu de méthionine à la position 87 de SEQ ID NO: 1 ou à la position 64 of SEQ ID NO: 2 ou à la position 56 of SEQ ID NO: 3 a été remplacé par norleucine ; éventuellement où :
(i) le résidu de lysine à la position 98 de SEQ ID NO: 1 ou à la position 75 of SEQ ID NO: 2 ou à la position 67 of SEQ ID NO: 3 est biotinylé via un groupe d'espaceur en polyéthylène glycol (PEG), afin de permettre l'immobilisation de la chimiokine sur un support solide, éventuellement où l'espaceur PEG est l'acide 3,6-dioxo aminooctanoïque ;
et / ou
(ii) la chimiokine MCP-1 modifiée ayant la structure montrée dans l'une quelconque des figures 7 à 9 ;
et / ou
(iii) la chimiokine MCP-1 modifiée est un agoniste ou un antagoniste d'activité de CCR2.

6. Réactif de liaison pour l'usage selon la revendication 1, où le réactif de liaison est une chimiokine CCL2 modifiée comprenant la séquence d'acide aminé telle que SEQ ID NO: 10 ou SEQ ID NO: 8, éventuellement où :
(i) le résidu à la position 75 est biotinylé, éventuellement via un groupe d'espaceur, afin de permettre l'immobilisation de la chimiokine sur un support solide, éventuellement où le groupe d'espaceur est un groupe d'espaceur de polyéthylène glycol (PEG), et éventuellement où l'espaceur PEG est l'acide 3,6-dioxo aminooctanoïque ;
et / ou
(ii) la chimiokine CCL2 modifiée est un agoniste ou un antagoniste d'activité de CCR2.


### SEQUENCE LISTING

<110> ITH Immune Therapy Holdings AB
<120> TREATING MULTIPLE SCLEROSIS
<130> P117759WO00
<160> 31
<170> PatentIn version 3.5
<210> 1
   <211> 99
   <212> PRT
   <213> Artificial sequence
<220>
   <223> Synthesised peptide
<400> 1
   Met Lys Val Ser Ala Ala Leu Leu Cys Leu Leu Leu Ile Ala Ala Thr
   1               5                   10                  15
   Phe Ile Pro Gln Gly Leu Ala Gln Pro Asp Ala Ile Asn Ala Pro Val
               20                  25                  30
   Thr Cys Cys Tyr Asn Phe Thr Asn Arg Lys Ile Ser Val Gln Arg Leu
           35                  40                  45
   Ala Ser Tyr Arg Arg Ile Thr Ser Ser Lys Cys Pro Lys Glu Ala Val
       50                  55                  60
   Ile Phe Lys Thr Ile Val Ala Lys Glu Ile Cys Ala Asp Pro Lys Gln
   65                  70                  75                  80
   Lys Trp Val Gln Asp Ser Met Asp His Leu Asp Lys Gln Thr Gln Thr
                   85                  90                  95
   Pro Lys Thr
<210> 2
   <211> 76
   <212> PRT
   <213> Artificial sequence
<220>
   <223> Synthesised peptide
<220>
   <221> X
   <222> (64)..(64)
   <223> X = Met or Nleu
<400> 2
   Gln Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Xaa
       50                  55                  60
   Asp His Leu Asp Lys Gln Thr Gln Thr Pro Lys Thr
   65                  70                  75
<210> 3
   <211> 68
   <212> PRT
   <213> Artificial sequence
<220>
   <223> Synthesised peptide
<400> 3
   Val Thr Cys Cys Tyr Asn Phe Thr Asn Arg Lys Ile Ser Val Gln Arg
   1               5                   10                  15
   Leu Ala Ser Tyr Arg Arg Ile Thr Ser Ser Lys Cys Pro Lys Glu Ala
               20                  25                  30
   Val Ile Phe Lys Thr Ile Val Ala Lys Glu Ile Cys Ala Asp Pro Lys
           35                  40                  45
   Gln Lys Trp Val Gln Asp Ser Met Asp His Leu Asp Lys Gln Thr Gln
       50                  55                  60
   Thr Pro Lys Thr
   65
<210> 4
   <211> 104
   <212> PRT
   <213> Artificial sequence
<220>
   <223> Synthesised peptide
<400> 4
   Met Lys Ile Ser Thr Leu Leu Cys Leu Leu Leu Ile Ala Thr Thr Ile
   1               5                   10                  15
   Ser Pro Gln Val Leu Ala Gly Pro Asp Ala Val Ser Thr Pro Val Thr
               20                  25                  30
   Cys Cys Tyr Asn Val Val Lys Gln Lys Ile His Val Arg Lys Leu Lys
           35                  40                  45
   Ser Tyr Arg Arg Ile Thr Ser Ser Gln Cys Pro Arg Glu Ala Val Ile
       50                  55                  60
   Phe Arg Thr Ile Leu Asp Lys Glu Ile Cys Ala Asp Pro Lys Glu Lys
   65                  70                  75                  80
   Trp Val Lys Asn Ser Ile Asn His Leu Asp Lys Thr Ser Gln Thr Phe
                   85                  90                  95
   Ile Leu Glu Pro Ser Cys Leu Gly
               100
<210> 5
   <211> 82
   <212> PRT
   <213> Artificial sequence
<220>
   <223> Synthesised peptide
<400> 5
   Gly Pro Asp Ala Val Ser Thr Pro Val Thr Cys Cys Tyr Asn Val Val
   1               5                   10                  15
   Lys Gln Lys Ile His Val Arg Lys Leu Lys Ser Tyr Arg Arg Ile Thr
               20                  25                  30
   Ser Ser Gln Cys Pro Arg Glu Ala Val Ile Phe Arg Thr Ile Leu Asp
           35                  40                  45
   Lys Glu Ile Cys Ala Asp Pro Lys Glu Lys Trp Val Lys Asn Ser Ile
       50                  55                  60
   Asn His Leu Asp Lys Thr Ser Gln Thr Phe Ile Leu Glu Pro Ser Cys
   65                  70                  75                  80
   Leu Gly
<210> 6
   <211> 76
   <212> PRT
   <213> Artificial sequence
<220>
   <223> Synthesised peptide
<400> 6
   Gly Pro Asp Ala Val Ser Thr Pro Val Thr Cys Cys Tyr Asn Val Val
   1               5                   10                  15
   Lys Gln Lys Ile His Val Arg Lys Leu Lys Ser Tyr Arg Arg Ile Thr
               20                  25                  30
   Ser Ser Gln Cys Pro Arg Glu Ala Val Ile Phe Arg Thr Ile Leu Asp
           35                  40                  45
   Lys Glu Ile Cys Ala Asp Pro Lys Glu Lys Trp Val Lys Asn Ser Ile
       50                  55                  60
   Asn His Leu Asp Lys Thr Ser Gln Thr Phe Ile Leu
   65                  70                  75
<210> 7
   <211> 76
   <212> PRT
   <213> Artificial sequence
<220>
   <223> Synthesised peptide
<400> 7
   Gly Pro Asp Ala Val Ser Thr Pro Val Thr Cys Cys Tyr Asn Val Val
   1               5                   10                  15
   Lys Gln Lys Ile His Val Arg Lys Leu Lys Ser Tyr Arg Arg Ile Thr
               20                  25                  30
   Ser Ser Gln Cys Pro Arg Glu Ala Val Ile Phe Arg Thr Ile Leu Asp
           35                  40                  45
   Lys Glu Ile Cys Ala Asp Pro Lys Glu Lys Trp Val Lys Asn Ser Ile
       50                  55                  60
   Asn His Leu Asp Lys Thr Ser Gln Thr Phe Lys Leu
   65                  70                  75
<210> 8
   <211> 76
   <212> PRT
   <213> Artificial sequence
<220>
   <223> Synthesised peptide
<220>
   <221> X
   <222> (1)..(1)
   <223> X = pyroGlu
<400> 8
   Xaa Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Lys Thr
   65                  70                  75
<210> 9
   <211> 76
   <212> PRT
   <213> Artificial sequence
<220>
   <223> Synthesised peptide
<220>
   <221> X
   <222> (1)..(1)
   <223> X = pyroGlu
<220>
   <221> X
   <222> (75)..(75)
   <223> X is K(ivDde)
<400> 9
   Xaa Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Xaa Thr
   65                  70                  75
<210> 10
   <211> 76
   <212> PRT
   <213> Artificial sequence
<220>
   <223> Synthesised peptide
<220>
   <221> X
   <222> (1)..(1)
   <223> X = pyroGlu
<220>
   <221> X
   <222> (75)..(75)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 10
   Xaa Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Xaa Thr
   65                  70                  75
<210> 11
   <211> 76
   <212> PRT
   <213> Artificial sequence
<220>
   <223> Synthesised peptide
<220>
   <221> X
   <222> (1)..(1)
   <223> X = pyroGlu
<400> 11
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Lys Pro
   65                  70                  75
<210> 12
   <211> 76
   <212> PRT
   <213> Artificial sequence
<220>
   <223> Synthesised peptide
<220>
   <221> X
   <222> (1)..(1)
   <223> X = pyroGlu
<220>
   <221> X
   <222> (75)..(75)
   <223> X is K(ivDde)
<400> 12
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Xaa Pro
   65                  70                  75
<210> 13
   <211> 76
   <212> PRT
   <213> Artificial sequence
<220>
   <223> Synthesised peptide
<220>
   <221> X
   <222> (1)..(1)
   <223> X = pyroGlu
<220>
   <221> X
   <222> (75)..(75)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 13
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Xaa Pro
   65                  70                  75
<210> 14
   <211> 74
   <212> PRT
   <213> Artificial sequence
<220>
   <223> Synthesised peptide
<220>
   <221> X
   <222> (73)..(73)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 14
   Gly Pro Ala Ser Val Pro Thr Thr Cys Cys Phe Asn Leu Ala Asn Arg
   1               5                   10                  15
   Lys Ile Pro Leu Gln Arg Leu Glu Ser Tyr Arg Arg Ile Thr Ser Gly
               20                  25                  30
   Lys Cys Pro Gln Lys Ala Val Ile Phe Lys Thr Lys Leu Ala Lys Asp
           35                  40                  45
   Ile Cys Ala Asp Pro Lys Lys Lys Trp Val Gln Asp Ser Met Lys Tyr
       50                  55                  60
   Leu Asp Gln Lys Ser Pro Thr Pro Xaa Pro
   65                  70
<210> 15
   <211> 74
   <212> PRT
   <213> Artificial sequence
<220>
   <223> Synthesised peptide
<220>
   <221> X
   <222> (73)..(73)
   <223> X is K(ivDde)
<400> 15
   Gly Pro Ala Ser Val Pro Thr Thr Cys Cys Phe Asn Leu Ala Asn Arg
   1 5 10 15
   Lys Ile Pro Leu Gln Arg Leu Glu Ser Tyr Arg Arg Ile Thr Ser Gly
   20 25 30
   Lys Cys Pro Gln Lys Ala Val Ile Phe Lys Thr Lys Leu Ala Lys Asp
   35 40 45
   Ile Cys Ala Asp Pro Lys Lys Lys Trp Val Gln Asp Ser Met Lys Tyr
   50 55 60
   Leu Asp Gln Lys Ser Pro Thr Pro Xaa Pro
   65 70
<210> 16
   <211> 74
   <212> PRT
   <213> Artificial sequence
<220>
   <223> Synthesised peptide
<220>
   <221> X
   <222> (73)..(73)
   <223> X is K(PEG-Biotin)
<400> 16
   Gly Pro Ala Ser Val Pro Thr Thr Cys Cys Phe Asn Leu Ala Asn Arg
   1 5 10 15
   Lys Ile Pro Leu Gln Arg Leu Glu Ser Tyr Arg Arg Ile Thr Ser Gly
   20 25 30
   Lys Cys Pro Gln Lys Ala Val Ile Phe Lys Thr Lys Leu Ala Lys Asp
   35 40 45
   Ile Cys Ala Asp Pro Lys Lys Lys Trp Val Gln Asp Ser Met Lys Tyr
   50 55 60
   Leu Asp Gln Lys Ser Pro Thr Pro Xaa Pro
   65 70
<210> 17
   <211> 68
   <212> PRT
   <213> Artificial sequence
<220>
   <223> Synthesised peptide
<400> 17
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Lys Ser
   65
<210> 18
   <211> 68
   <212> PRT
   <213> Artificial sequence
<220>
   <223> Synthesised peptide
<220>
   <221> X
   <222> (67)..(67)
   <223> X is K(ivDde)
<400> 18
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Xaa Ser
   65
<210> 19
   <211> 68
   <212> PRT
   <213> Artificial sequence
<220>
   <223> Synthesised peptide
<220>
   <221> X
   <222> (67)..(67)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 19
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Xaa Ser
   65
<210> 20
   <211> 70
   <212> PRT
   <213> Artificial sequence
<220>
   <223> Synthesised peptide
<400> 20
   Ala Ser Asn Phe Asp Cys Cys Leu Gly Tyr Thr Asp Arg Ile Leu His
   1               5                   10                  15
   Pro Lys Phe Ile Val Gly Phe Thr Arg Gln Leu Ala Asn Glu Gly Cys
               20                  25                  30
   Asp Ile Asn Ala Ile Ile Phe His Thr Lys Lys Lys Leu Ser Val Cys
           35                  40                  45
   Ala Asn Pro Lys Gln Thr Trp Val Lys Tyr Ile Val Arg Leu Leu Ser
       50                  55                  60
   Lys Lys Val Lys Asn Met
   65                  70
<210> 21
   <211> 70
   <212> PRT
   <213> Artificial sequence
<220>
   <223> Synthesised peptide
<220>
   <221> X
   <222> (68)..(68)
   <223> X is K(ivDde)
<400> 21
   Ala Ser Asn Phe Asp Cys Cys Leu Gly Tyr Thr Asp Arg Ile Leu His
   1               5                   10                  15
   Pro Lys Phe Ile Val Gly Phe Thr Arg Gln Leu Ala Asn Glu Gly Cys
               20                  25                  30
   Asp Ile Asn Ala Ile Ile Phe His Thr Lys Lys Lys Leu Ser Val Cys
           35                  40                  45
   Ala Asn Pro Lys Gln Thr Trp Val Lys Tyr Ile Val Arg Leu Leu Ser
       50                  55                  60
   Lys Lys Val Xaa Asn Met
   65                  70
<210> 22
   <211> 70
   <212> PRT
   <213> Artificial sequence
<220>
   <223> Synthesised peptide
<220>
   <221> X
   <222> (68)..(68)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 22
   Ala Ser Asn Phe Asp Cys Cys Leu Gly Tyr Thr Asp Arg Ile Leu His
   1 5 10 15
   Pro Lys Phe Ile Val Gly Phe Thr Arg Gln Leu Ala Asn Glu Gly Cys
   20 25 30
   Asp Ile Asn Ala Ile Ile Phe His Thr Lys Lys Lys Leu Ser Val Cys
   35 40 45
   Ala Asn Pro Lys Gln Thr Trp Val Lys Tyr Ile Val Arg Leu Leu Ser
   50 55 60
   Lys Lys Val Xaa Asn Met
   65 70
<210> 23
   <211> 74
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (64)..(64)
   <223> X = Norleucine
<400> 23
   Xaa Gly Val Phe Glu Asp Cys Cys Leu Ala Tyr His Tyr Pro Ile Gly
   1 5 10 15
   Trp Ala Val Leu Arg Arg Ala Trp Thr Tyr Arg Ile Gln Glu Val Ser
   20 25 30
   Gly Ser Cys Asn Leu Pro Ala Ala Ile Phe Tyr Leu Pro Lys Arg His
   35 40 45
   Arg Lys Val Cys Gly Asn Pro Lys Ser Arg Glu Val Gln Arg Ala Xaa
   50 55 60
   Lys Leu Leu Asp Ala Arg Asn Lys Val Phe
   65 70
<210> 24
   <211> 74
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (64)..(64)
   <223> X = Norleucine
<220>
   <221> X
   <222> (72)..(72)
   <223> X is K(ivDde)
<400> 24
   Xaa Gly Val Phe Glu Asp Cys Cys Leu Ala Tyr His Tyr Pro Ile Gly
   1               5                   10                  15
   Trp Ala Val Leu Arg Arg Ala Trp Thr Tyr Arg Ile Gln Glu Val Ser
               20                  25                  30
   Gly Ser Cys Asn Leu Pro Ala Ala Ile Phe Tyr Leu Pro Lys Arg His
           35                  40                  45
   Arg Lys Val Cys Gly Asn Pro Lys Ser Arg Glu Val Gln Arg Ala Xaa
       50                  55                  60
   Lys Leu Leu Asp Ala Arg Asn Xaa Val Phe
   65                  70
<210> 25
   <211> 74
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (64)..(64)
   <223> X = Norleucine
<220>
   <221> X
   <222> (72)..(72)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 25
   Xaa Gly Val Phe Glu Asp Cys Cys Leu Ala Tyr His Tyr Pro Ile Gly
   1               5                   10                  15
   Trp Ala Val Leu Arg Arg Ala Trp Thr Tyr Arg Ile Gln Glu Val Ser
               20                  25                  30
   Gly Ser Cys Asn Leu Pro Ala Ala Ile Phe Tyr Leu Pro Lys Arg His
           35                  40                  45
   Arg Lys Val Cys Gly Asn Pro Lys Ser Arg Glu Val Gln Arg Ala Xaa
       50                  55                  60
   Lys Leu Leu Asp Ala Arg Asn Xaa Val Phe
   65                  70
<210> 26
   <211> 73
   <212> PRT
   <213> Artificial sequence
<220>
   <223> Synthesised peptide
<400> 26
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe
   65                  70
<210> 27
   <211> 74
   <212> PRT
   <213> Artificial sequence
<220>
   <223> Synthesised peptide
<220>
   <221> X
   <222> (74)..(74)
   <223> X is PEG-K(ivDde)
<400> 27
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe Xaa
   65                  70
<210> 28
   <211> 74
   <212> PRT
   <213> Artificial sequence
<220>
   <223> Synthesised peptide
<220>
   <221> X
   <222> (74)..(74)
   <223> X = residue that can be biotinylated, e.g. lysine, ornithine or diaminopropionic acid and optionally is biotinylated, optionally via a spacer molecule such as PEG, e.g. K(PEG-Biotin) and may be attached via a spacer molecule, e.g.PEG-K(Biotin)
<400> 28
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe Xaa
   65                  70
<210> 29
   <211> 77
   <212> PRT
   <213> Artificial sequence
<220>
   <223> Synthesised peptide
<400> 29
   Val Pro Leu Ser Arg Thr Val Arg Cys Thr Cys Ile Ser Ile Ser Asn
   1               5                   10                  15
   Gln Pro Val Asn Pro Arg Ser Leu Glu Lys Leu Glu Ile Ile Pro Ala
               20                  25                  30
   Ser Gln Phe Cys Pro Arg Val Glu Ile Ile Ala Thr Met Lys Lys Lys
           35                  40                  45
   Gly Glu Lys Arg Cys Leu Asn Pro Glu Ser Lys Ala Ile Lys Asn Leu
       50                  55                  60
   Leu Lys Ala Val Ser Lys Glu Arg Ser Lys Arg Ser Pro
   65                  70                  75
<210> 30
   <211> 78
   <212> PRT
   <213> Artificial sequence
<220>
   <223> Synthesised peptide
<220>
   <221> X
   <222> (77)..(77)
   <223> X is K(ivDde), optionally attached via a spacer such as PEG, e.g. -PEG-K(ivDde)
<220>
   <221> misc_feature
   <222> (78)..(78)
   <223> Xaa can be any naturally occurring amino acid
<400> 30
   Val Pro Leu Ser Arg Thr Val Arg Cys Thr Cys Ile Ser Ile Ser Asn
   1               5                   10                  15
   Gln Pro Val Asn Pro Arg Ser Leu Glu Lys Leu Glu Ile Ile Pro Ala
               20                  25                  30
   Ser Gln Phe Cys Pro Arg Val Glu Ile Ile Ala Thr Met Lys Lys Lys
           35                  40                  45
   Gly Glu Lys Arg Cys Leu Asn Pro Glu Ser Lys Ala Ile Lys Asn Leu
       50                  55                  60
   Leu Lys Ala Val Ser Lys Glu Arg Ser Lys Arg Ser Pro Xaa
   65                  70                  75
<210> 31
   <211> 78
   <212> PRT
   <213> Artificial sequence
<220>
   <223> Synthesised peptide
<220>
   <221> X
   <222> (77)..(77)
   <223> X = residue that can be biotinylated, e.g. lysine, ornithine or diaminopropionic acid and optionally is biotinylated, optionally via a spacer molecule such as PEG, e.g. K(PEG-Biotin) and may be attached via a spacer molecule, e.g.PEG-K(Biotin)
<220>
   <221> misc_feature
   <222> (78)..(78)
   <223> Xaa can be any naturally occurring amino acid
<400> 31
   Val Pro Leu Ser Arg Thr Val Arg Cys Thr Cys Ile Ser Ile Ser Asn
   1               5                   10                  15
   Gln Pro Val Asn Pro Arg Ser Leu Glu Lys Leu Glu Ile Ile Pro Ala
               20                  25                  30
   Ser Gln Phe Cys Pro Arg Val Glu Ile Ile Ala Thr Met Lys Lys Lys
           35                  40                  45
   Gly Glu Lys Arg Cys Leu Asn Pro Glu Ser Lys Ala Ile Lys Asn Leu
       50                  55                  60
   Leu Lys Ala Val Ser Lys Glu Arg Ser Lys Arg Ser Pro Xaa
   65                  70                  75